# EUROPEAN PATENT APPLICATION

(11) **EP 4 653 434 A1**
(43) Date of publication of application: **26.11.2025**
(21) Application number: 24178115.2
(22) Date of filing: 25.05.2024
(51) Int. Cl.: C07D 413/14, C07C 53/18

(54) **CRYSTALLINE SALT OF MACROPA-(OCH2CH2)-PH-NCS**

(71) Applicant: Bayer Aktiengesellschaft, 51373 Leverkusen (DE)
(72) Inventor: The designation of the inventor has not yet been filed
(74) Representative: BIP Patents

(57) **Abstract**

The present disclosure relates to the trifluoroacetic salt of a compound of formula (I), a crystalline form thereof, a process for its preparation and its use.

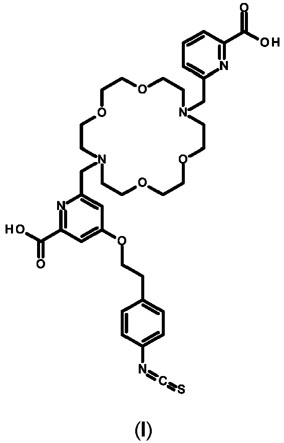

## Description

### FIELD OF THE INVENTION

The present disclosure relates to the items characterized in the patent claims, i.e. to the trifluoroacetic salt of a compound of formula (I), a crystalline form thereof, a process for its preparation and its use.

### BACKGROUND

### 1. Introduction

The macrocyclic chelator macropa (6-[[16-[(6-carboxypyridin-2-yl)methyl]-1,4,10,13-tetraoxa-7,16-diazacyclooctadec-7-yl]methyl]pyridine-2-carboxylic acid) has been investigated due to its radionuclide-binding properties and its potential application in conjugates with small molecules, monoclonal antibodies or other targeting moieties which can be useful for radiotherapy (see, for example: McDevitt et al., Pharmaceuticals 2024, 17(1), 76, https://doi.org/10.3390/ph17010076 and Mamat et al., Inorg. Chem. 2023, 62(50), 20699, https://doi.org/10.1021/acs.inorgchem.3c01983). One of the ways of incorporating the chelating moiety into a drug conjugate is by coupling a macropa derivative comprising an isothiocyanate moiety such as macropa-(OCH2CH2)-Ph-NCS of formula (I) 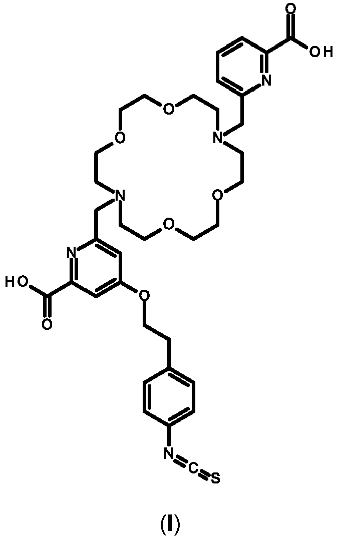 with an amino group of the desired targeting moiety via a thiourea group.

### 2. Description of the prior art, problem to be solved and its solution

WO2020/106886 describes the synthesis of macropa-(OCH2CH2)-Ph-NCS of formula (I) comprising a final activation step of macropa-(OCH2CH2)-Ph-NH2 of formula (II) using di-2-pyridyl-thionocarbonate in a mixture of water and acetonitrile in the presence of triethylamine followed by direct purification by chromatography (Scheme 1).

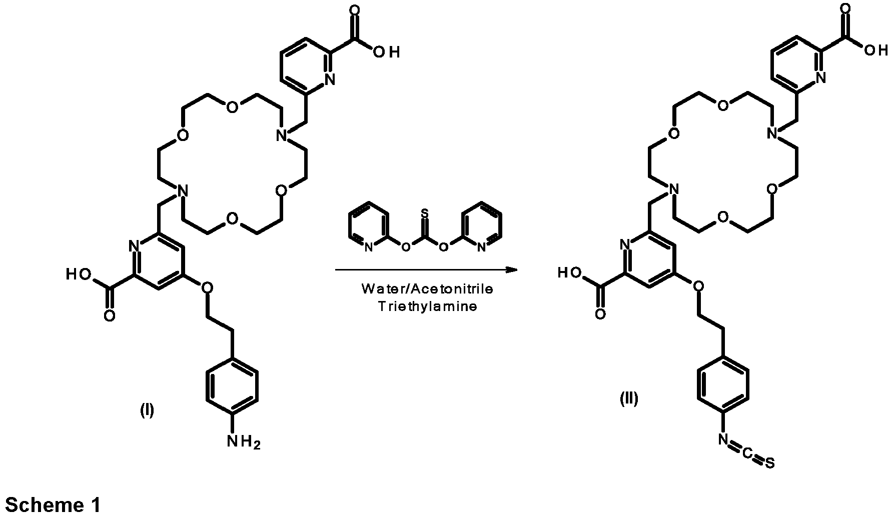

The conversion of macropa-(OCH2CH2)-Ph-NH2 in a mixture of water and acetonitrile - even without the use of a base - is a rapid reaction. However, due to the degradation of macropa-(OCH2CH2)-Ph-NCS under the isothiocyanate-forming reaction conditions, immediate purification of the product is essential. Further, alkaline aqueous conditions accelerate the hydrolysis of the isothiocyanate, but very few alternatives to the use of aqueous solutions are possible in view of the low solubility of macropa-(OCH2CH2)-Ph-NH2 in non-aqueous solvents.

The most prominent degradation product is the symmetrical bis-macropa thiourea (Scheme 2) which results from the addition of the initial hydrolysis product macropa-(OCH2CH2)-Ph-NH2 to the remaining macropa-(OCH2CH2)-Ph-NCS. Therefore, direct purification by chromatography, i.e., reversed phase chromatography, is essential. Isolated macropa-(OCH2CH2)-Ph-NCS is obtained as an amorphous material upon lyophilization/freeze drying.

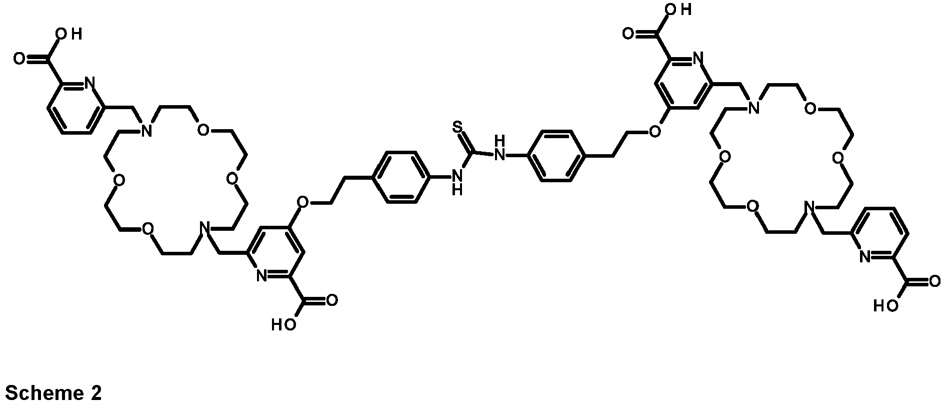

The isolated amorphous macropa-(OCH2CH2)-Ph-NCS has a poor stability and undergoes a rapid decline in quality upon storage at room temperature. Further, amorphous macropa-(OCH2CH2)-Ph-NCS is still insufficiently stable upon storage at < -18°C. Indeed, the main degradation product, i.e., the symmetrical bis-macropa-thiourea already forms during the freeze-drying (lyophilization) of the chromatography product fractions. Finally, amorphous materials tend to be generally hygroscopic and therefore the risk for degradation is intrinsic. This is especially worrying in the case of macropa-(OCH2CH2)-Ph-NCS, which is particularly sensitive to hydrolysis, thus severely complicating its use in the preparation of conjugates suitable for pharmaceutical application.

For an application in antibody conjugation, for example, it is highly desirable and advantageous that such a material be water-soluble in order to avoid the use of organic cosolvents during the conjugation reaction. The use of organic cosolvents in antibody conjugations is a known risk for antibody aggregation and antibody aggregates represent a known immunogenicity risk. In addition, purely aqueous processes have a significantly lower potential of contamination via leachables & extractables.

Thus, the synthesis of macropa-(OCH2CH2)-Ph-NCS disclosed in the prior art is therefore not suited and highly disadvantageous for scale-up and therefore for the purposes of producing and supplying the large quantities of material that the the manufacturing of a commercial product (e.g., a radionuclide-containing conjugate) requires. The stability issues of amorphous macropa-(OCH2CH2)-Ph-NCS are of particular concern from a logistical perspective, since they would be a limiting factor constraining the reliable preparation and delivery of any macropa-containing drug product across countries and/or continents.

Thus, there is an unmet need to provide macropa-(OCH2CH2)-Ph-NCS which is sufficiently stable over long periods of time such that it allows its reliable use in the development and manufacture of drug products. Further, there is an unmet need to provide processes leading to stable forms of macropa-(OCH2CH2)-Ph-NCS which are suitable for scale-up and which can reliably provide sufficient quantities of the material for the production and delivery of any macropa-containing drug product. In particular, there is an unmet need for a robust and reproducible process that allows for the manufacture of a stable form of macropa-(OCH2CH2)-Ph-NCS in a scale which is suitable to deliver a high-quality material that is sufficiently stable for prolonged storage and to supply the market demands of macropa-containing conjugates such as conjugates with monoclonal antibodies, small molecules or peptidic moieties.

The present disclosure solves these issues by providing (i) the trifluoroacetic acid salt of macropa-(OCH2CH2)-Ph-NCS of formula (I), (ii) a crystalline form of the trifluoroacetic acid salt of macropa-(OCH2CH2)-Ph-NCS of formula (I) as well as (iii) processes for the preparation of both the trifluoroacetic acid salt of macropa-(OCH2CH2)-Ph-NCS of formula (I) and of a crystalline form of the trifluoroacetic acid salt of macropa-(OCH2CH2)-Ph-NCS of formula (I).

It has surprisingly been found that the use of trifluoroacetic acid is advantageous in solubilizing macropa-(OCH2CH2)-Ph-NH2, i.e., the most suitable starting material for the preparation of macropa-(OCH2CH2)-Ph-NCS of formula (I) even in solvents which would typically lead to the formation of carbamate byproducts (alcohols) while at the same time providing a salt and particularly a crystalline salt of macropa-(OCH2CH2)-Ph-NCS of formula (I) which can be easily prepared under mild reaction conditions and conveniently isolated in good yields.

It has surprisingly been found that the trifluoroacetic acid salt of macropa-(OCH2CH2)-Ph-NCS of formula (I) and particularly the crystalline form of the trifluoroacetic acid salt of macropa-(OCH2CH2)-Ph-NCS of formula (I) of the present disclosure display a significantly enhanced stability and a remarkable reduction and/or absence of hygroscopicity in comparison to the amorphous lyophilizate of the free base of macropa-(OCH2CH2)-Ph-NCS. In addition, the stable forms of macropa-(OCH2CH2)-Ph-NCS of the present disclosure show an increased solubility in water which is advantageous for conjugation, particularly for antibody and peptide conjugation.

Further, the processes for the preparation of the trifluoroacetic acid salt of macropa-(OCH2CH2)-Ph-NCS of formula (I) and of the crystalline form of the trifluoroacetic acid salt of macropa-(OCH2CH2)-Ph-NCS of formula (I) are suitable for scale-up and deliver the stable forms of macropa-(OCH2CH2)-Ph-NCS of formula (I) in high quantities and good yields. Further, the processes of the present disclosure avoid the use of aqueous conditions in the preparation of the stable forms of macropa-(OCH2CH2)-Ph-NCS of formula (I), thus reducing the risk of hydrolysis and of the formation of undesired by-products. Despite the low solubility of the starting material macropa-(OCH2CH2)-Ph-NH2 of formula (II) in non-aqueous solvents, the processes of the present disclosure can be carried out avoiding the use of aqueous media and strike the balance between ensuring a sufficient solubility of the starting material and avoiding the formation of undesired by-products due to the reactivity of the isothiocyanate group in macropa-(OCH2CH2)-Ph-NCS.

### DESCRIPTION OF THE INVENTION

The present disclosure relates to the trifluoroacetic acid salt of a compound of formula (I), 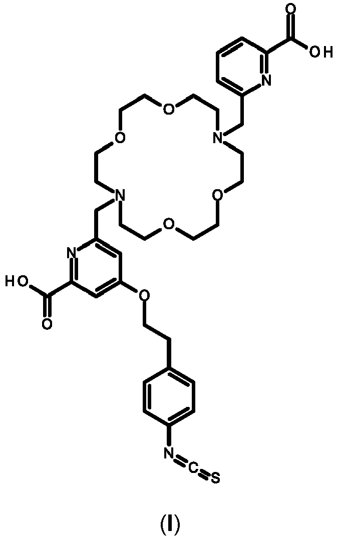 to a crystalline form of the trifluoroacetic acid salt of a compound of formula (I), to processes for the preparation of the trifluoroacetic acid salt of a compound of formula (I) and for the preparation of a crystalline form of the trifluoroacetic acid salt of a compound of formula (I) and to the use of the trifluoroacetic acid salt of a compound of formula (I) and of a crystalline form of the trifluoroacetic acid salt of a compound of formula (I).

### DEFINITIONS

In the context of the present disclosure, the compound of formula (I) 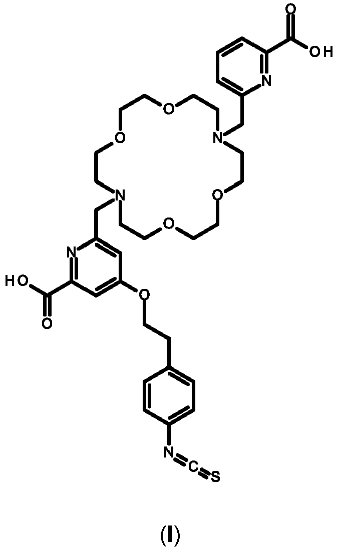 may also be referred to as macropa-(OCH2CH2)-Ph-NCS.

In the context of the present disclosure, the compound of formula (II) 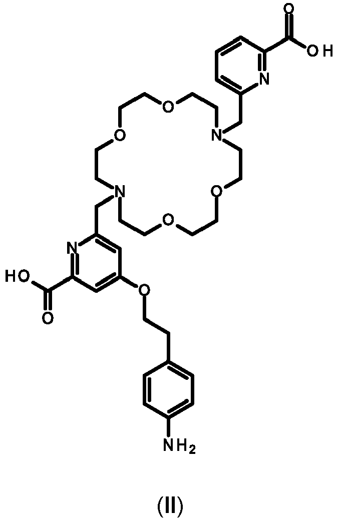 may also be referred to as macropa-(OCH2CH2)-Ph-NH2.

In the context of the present disclosure, the term "the trifluoroacetic acid salt of a compound of formula (I)" or "a trifluoroacetic acid salt of a compound of formula (I)" refers to a salt comprising one molar equivalent of the compound of formula (I) and two molar equivalents of trifluoroacetic acid, i.e., to the bis-trifluoroacetate salt of the compound of formula (I), i.e. to macropa-(OCH2CH2)-Ph-NCS x 2 TFA as depicted below:

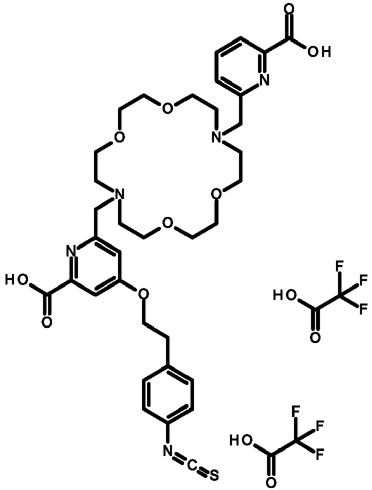

In the context of the present disclosure, the term "the crystalline trifluoroacetic acid salt of a compound of formula (I)" or "a crystalline trifluoroacetic acid salt of a compound of formula (I)" refers to a crystalline salt comprising one molar equivalent of the compound of formula (I) and two molar equivalents of trifluoroacetic acid, i.e., to the crystalline bis-trifluoroacetate salt of the compound of formula (I), i.e., to crystalline macropa-(OCH2CH2)-Ph-NCS x 2 TFA, i.e. to a (the) crystalline form of macropa-(OCH2CH2)-Ph-NCS x 2 TFA as depicted below:

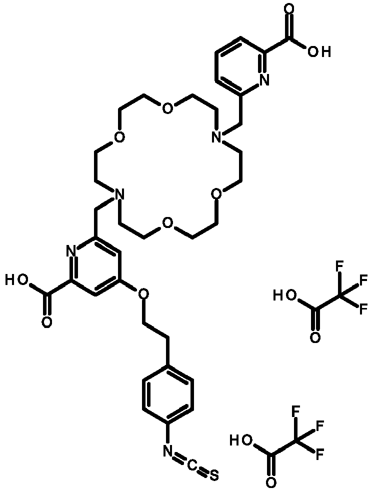

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero.

When groups in the compounds according to the invention are substituted, it is possible for said groups to be mono-substituted or poly-substituted with substituent(s), unless otherwise specified. Within the scope of the present invention, the meanings of all groups which occur repeatedly are independent from one another. It is possible that groups in the compounds according to the invention are substituted with one, two or three identical or different substituents, particularly with one substituent.

The term "comprising" when used in the specification includes "consisting of" and "consisting essentially of".

If within the present disclosure any item is referred to as "as mentioned herein", it means that it may be mentioned anywhere in the present disclosure.

In the context of the present disclosure, the term "room temperature" refers to a temperature in the range of from 20 to 25°C, preferably to a temperature of 23°C.

The term "C1-C3", as used in the present disclosure, *e.g*. in the context of the definition of "C1-C3 alcohol" means an alkyl group having a finite number of carbon atoms of 1 to 3, *i.e.* 1, 2 or 3 carbon atoms. The term "C1-C3 alcohol" as used within the present disclosure refers to a linear or branched alkyl chain having 1, 2 or 3 carbon atoms where at least one hydrogen atom is replaced with a hydrohy (OH) group.

The term "C5-C7 alkane" as used in the present disclosure, refers to a linear or branched alkyl chain having 5, 6 or 7 carbon atoms.

The term "C3-C5 alcohol", as used in the present disclosure, refers to a linear or branched alkyl chain having 3, 4 or 5 carbon atoms where at least one hydrogen atom is replaced with a hydroxy (OH) group.

The term "C2-C5 fluorinated alcohol containing at least three fluorine atoms", as used in the present disclosure, refers to a linear or branched alkyl chain having 2, 3, 4 or 5 carbon atoms where at least one hydrogen atom is replaced with a hydroxy (OH) group and where at least three further hydrogen atoms are replaced with fluorine atoms.

### DETAILED DESCRIPTION

The present disclosure relates to the trifluoroacetic acid salt of a compound of formula (I),

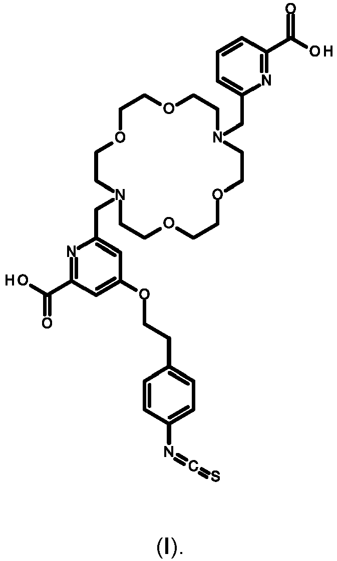

In one embodiment, the trifluoroacetic acid salt of a compound of formula (I) is in amorphous form.

### Crystalline form of the trifluoroacetic acid salt of a compound of formula (I)

In a further embodiment, the trifluoroacetic acid salt of a compound of formula (I) is crystalline. Thus, in a further embodiment, the present disclosure relates to the trifluoroacetic acid salt of a compound of formula (I) in crystalline form, i.e., to a crystalline form of the trifluoroacetic acid salt of a compound of formula (I), i.e., to a crystalline form of the trifluoroacetic acid salt of macropa-(OCH2CH2)-Ph-NCS of formula (I).

The crystalline form of the trifluoroacetic acid salt of a compound of formula (I) may be characterized by analytical methods well known in the field of the pharmaceutical industry for characterizing solids. Such methods comprise but are not limited to powder X-ray diffraction (PXRD/XRPD), Fourier transform Infrared (FTIR) spectroscopy, differential scanning calorimetry (DSC), thermogravimetric analysis (TGA) and dynamic vapour sorption (DVS). The crystalline form of the trifluoroacetic acid salt of a compound of formula (I) may be characterized by one of the aforementioned methods or by combining two or more of them. In particular, crystalline form of the trifluoroacetic acid salt of a compound of formula (I) may be characterized by one of the following embodiments or by combining two or more of the following embodiments.

The crystalline form of the trifluoroacetic acid salt of a compound of formula (I) is characterized by having a powder X-ray diffractogram comprising reflections at 2-Theta angles of (10.1 ± 0.2)°, (20.7 ± 0.2)° and (25.5 ± 0.2)°, when measured at room temperature with Cu-Kalpha1 radiation having a wavelength of 0.15419 nm. Preferably, the crystalline form of the trifluoroacetic acid salt of a compound of formula (I) is characterized by having a powder X-ray diffractogram comprising reflections at 2-Theta angles of (6.8 ± 0.2)°, (10.1 ± 0.2)°, (20.2 ± 0.2)°, (20.7 ± 0.2)° and (25.5 ± 0.2)°, when measured at room temperature with Cu-Kalpha1 radiation having a wavelength of 0.15419 nm. Preferably, the crystalline form of the trifluoroacetic acid salt of a compound of formula (I) is characterized by having a powder X-ray diffractogram comprising reflections at 2-Theta angles of (6.8 ± 0.2)°, (10.1 ± 0.2)°, (17.2 ± 0.2)°, (20.2 ± 0.2)°, (20.7 ± 0.2)°, (21.5 ± 0.2)° and (25.5 ± 0.2)°, when measured at room temperature with Cu-Kalpha1 radiation having a wavelength of 0.15419 nm. Preferably, the crystalline form of the trifluoroacetic acid salt of a compound of formula (I) is characterized by having a powder X-ray diffractogram comprising reflections at 2-Theta angles of (6.8 ± 0.2)°, (9.4 ± 0.2)°, (10.1 ± 0.2)°, (11.0 ± 0.2)°, (17.2 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm.

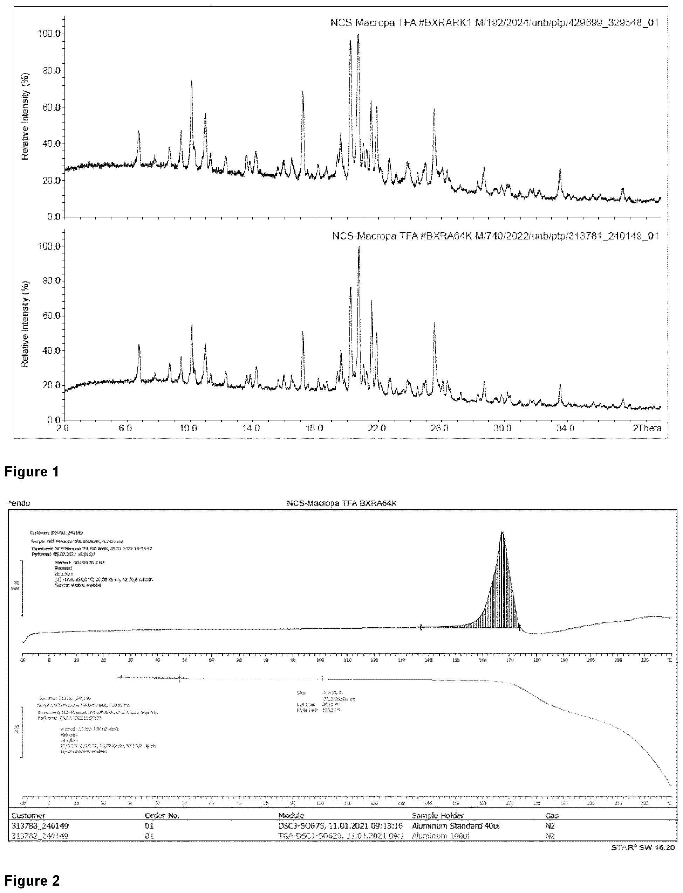

Alternatively or additionally, the crystalline form of the trifluoroacetic acid salt of a compound of formula (I) is characterized by having a powder X-ray diffractogram essentially the same as displayed in Figure 1 (top or bottom) of the present invention, when measured at room temperature with Cu-Kalpha1 radiation having a wavelength of 0.15419 nm.

Alternatively or additionally, the crystalline form of the trifluoroacetic acid salt of a compound of formula (I) is characterized by having a Fourier transform Infrared spectrum comprising bands at wavenumbers of (720 ± 2) cm-1, (1114 ± 2) cm-1, and (1170 ± 2) cm-1, when measured at room temperature with a diamond ATR cell. Preferably, the crystalline form of the trifluoroacetic acid salt of a compound of formula (I) is characterized by having a Fourier transform Infrared spectrum comprising peaks at wavenumbers of (720 ± 2) cm-1, (1036 ± 2) cm-1, (1114 ± 2) cm-1, (1170 ± 2) cm-1 and (1345 ± 2) cm-1, when measured at room temperature with a diamond ATR cell. Preferably, the crystalline form of the trifluoroacetic acid salt of a compound of formula (I) is characterized by having a Fourier transform Infrared spectrum comprising bands at wavenumbers of (720 ± 2) cm-1, (835 ± 2) cm-1, (1036 ± 2) cm-1, (1114 ± 2) cm-1, (1170 ± 2) cm-1, (1345 ± 2) cm-1 and (1673 ± 2) cm-1, when measured at room temperature with a diamond ATR cell. Preferably, the crystalline form of the trifluoroacetic acid salt of a compound of formula (I) is characterized by having a Fourier transform Infrared spectrum comprising bands at wavenumbers of (720 ± 2) cm-1, (779 ± 2) cm-1, (835 ± 2) cm-1, (1036 ± 2) cm-1, (1114 ± 2) cm-1, (1170 ± 2) cm-1, (1345 ± 2) cm-1, (1404 ± 2) cm-1, (1573 ± 2) cm-1 and (1673 ± 2) cm-1, when measured at room temperature with a diamond ATR cell. Preferably, the crystalline form of the trifluoroacetic acid salt of a compound of formula (I) is characterized by having a Fourier transform Infrared spectrum comprising bands at wavenumbers of (720 ± 2) cm-1, (779 ± 2) cm-1, (835 ± 2) cm-1, (1036 ± 2) cm-1, (1114 ± 2) cm-1, (1170 ± 2) cm-1, (1345 ± 2) cm-1, (1404 ± 2) cm-1, (1573 ± 2) cm-1, (1673 ± 2) cm-1, (1754 ± 2) cm-1 and (2124 ± 2) cm-1 when measured at room temperature with a diamond ATR cell.

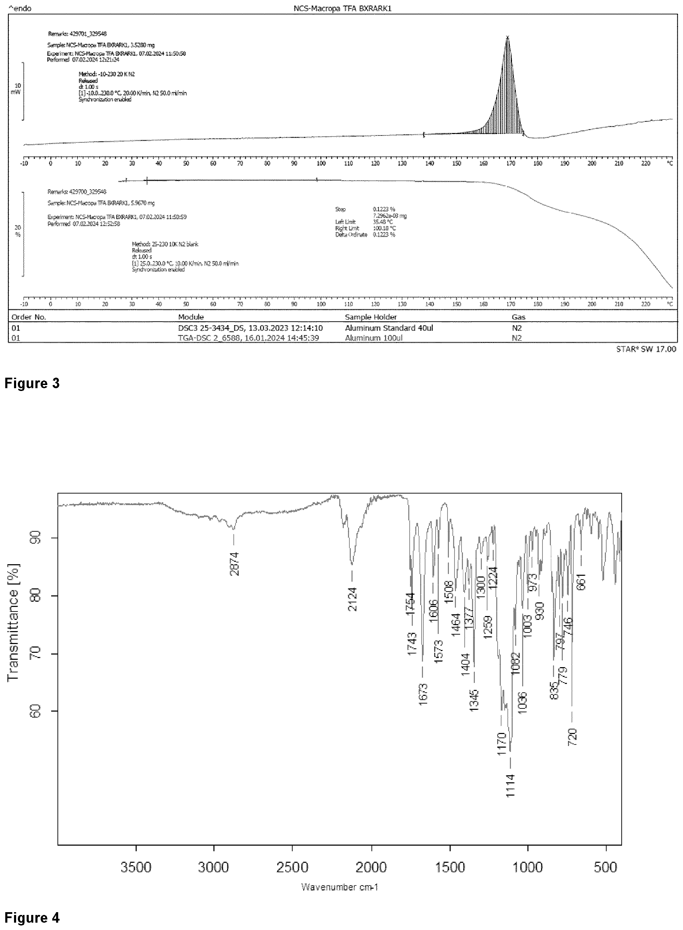

Alternatively, the crystalline form of the trifluoroacetic acid salt of a compound of formula (I) is characterized by having a Fourier transform Infrared spectrum essentially the same as displayed in Figure 4 of the present invention, when measured at room temperature with a diamond ATR cell.

### Process for the preparation of the trifluoroacetic acid salt of a compound of formula (I)

The present disclosure also relates to a process for the preparation of the trifluoroacetic acid salt of a compound of formula (I).

A compound of formula (II) 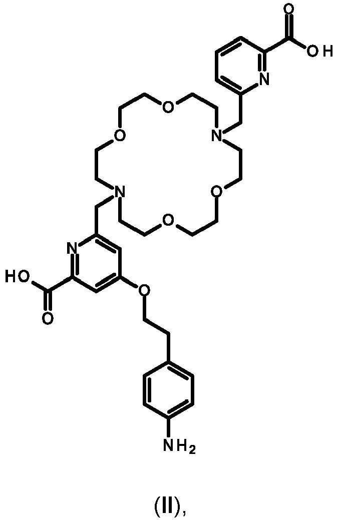 i.e., macropa-(OCH2CH2)-Ph-NH2, is a suitable starting material for the preparation of the trifluoroacetic acid salt of a compound of formula (I).

For the preparation of the trifluoroacetic acid salt of a compound of formula (I), the compound of formula (II) is first converted to the compound of formula (I). This conversion can be carried out in any suitable solvent or solvent mixture. Preferably, the conversion of the compound of formula (II) to the compound of formula (I) is carried out in a solvent or solvent mixture comprising water, acetonitrile, dichloromethane, tetrahydrofurane, 1-methoxy-2-propanol, sulfolane, acetic acid, dimethylacetamide, dimethyl sulfoxide, N-methyl pyrrolidone, dimethyl formamide, 1-propanol, 2-propanol, 1-butanol, 2-butanol, hexafluoro-2-propanol and trifluoroethanol or a mixture of two or more thereof. Preferably, the conversion of the compound of formula (II) to the compound of formula (I) is carried out in a solvent mixture comprising an organic solvent and water. When the conversion of the compound of formula (II) to the compound of formula (I) is carried out in a solvent mixture comprising an organic solvent and water, the organic solvent is preferably selected from acetonitrile, tetrahydrofurane, 1-methoxy-2-propanol, sulfolane, acetic acid, dimethylacetamide, dimethyl sulfoxide, N-methyl pyrrolidone, dimethyl formamide, 1-propanol, 2-propanol, 1-butanol, 2-butanol, hexafluoro-2-propanol and trifluoroethanol or a mixture of two or more thereof. More preferably, the conversion of the compound of formula (II) to the compound of formula (I) is carried out in a mixture of water and acetonitrile.

The conversion of the compound of formula (II) to the compound of formula (I) is preferably carried out in the absence of water. More preferably, the conversion of the compound of formula (II) to the compound of formula (I) is carried out in a solvent or solvent mixture comprising acetonitrile, dichloromethane, tetrahydrofurane, dimethylacetamide, dimethyl sulfoxide, N-methyl pyrrolidone, dimethyl formamide, 1-methoxy-2-propanol, 1-propanol, 2-propanol, 1-butanol, 2-butanol, hexafluoro-2-propanol and trifluoroethanol or a mixture of two or more thereof. More preferably, the conversion of the compound of formula (II) to the compound of formula (I) is carried out in an organic solvent selected from acetonitrile, dichloromethane, tetrahydrofurane, dimethylacetamide, dimethyl sulfoxide, N-methyl pyrrolidone, dimethyl formamide, 1-methoxy-2-propanol, 1-propanol, 2-propanol, 1-butanol, 2-butanol, hexafluoro-2-propanol and trifluoroethanol or a mixture of two or more thereof. More preferably, the conversion of the compound of formula (II) to the compound of formula (I) is carried out in an organic solvent selected from dimethylacetamide, dimethyl sulfoxide, N-methyl pyrrolidone, dimethyl formamide, 1-methoxy-2-propanol and hexafluoro-2-propanol or a mixture of two or more thereof. More preferably, the conversion of the compound of formula (II) to the compound of formula (I) is carried out in an organic solvent selected from acetonitrile, 2-propanol and hexafluoro-2-propanol or a mixture of two or more thereof. More preferably, the conversion of the compound of formula (II) to the compound of formula (I) is carried out in 2-propanol or in a mixture of 2-propanol and hexafluoro-2-propanol..

Depending on the nature of the compound of formula (II) employed, additional reagents (e.g. a base such as an amine base, a tertiary amine base such as triethylamine, diisopropylethylamine and the like or a heterocyclic base such as pyridine) may be necessary for the conversion of the compound of formula (II) to the compound of formula (I).

Any isothiocyanate-forming reagent can be used as long as it is suitable for the conversion of the amino group in the compound of formula (II) into the isothiocyanate group in the compound of formula (I). Preferably, the isothiocyanate-forming group is an aryl or heteroaryl thionocarbonate, an aryl or heteroaryl thiocarbonyl compound or thiophosgene. More preferably, the isothiocyanate-forming group is an aryl or heteroaryl thionocarbonate, thiocarbonyldiimidazol (di-1H-imidazol-1-ylmethanethione), bis(1H-1,2,4-triazol-1-yl)methanethione, bis(1H-benzotriazol-1-yl)methanethione or thiophosgene. More preferably, the isothiocyanate-forming group is bis(2-pyridyl)thionocarbonate, thiocarbonyldiimidazol (di-1H-imidazol-1-ylmethanethione), bis(1H-1,2,4-triazol-1-yl)methanethione, bis(1H-benzotriazol-1-yl)methanethione or thiophosgene. More preferably, the isothiocyanate-forming group is bis(2-pyridyl)thionocarbonate (CAS-No.: 96989-50-3).

The process can be carried out at any suitable temperature which allows for the conversion of the amine group in the compound of formula (II) to the isothiocyanate group in the compound of formula (I). Preferably, the reaction is carried out at a temperature of from 20°C to 40°C. More preferably the reaction is carried out at room temperature.

Once formed, the compound of formula (I) can be converted in situ to its trifluoroacetic acid salt, i.e., the trifluoroacetic acid salt of the compound of formula (I) by the addition of trifluoroacetic acid. Preferably, the trifluoroacetic acid salt of a compound of formula (I) is formed by purification by reversed-phase chromatography with at least one eluent comprising trifluoroacetic acid.

In one embodiment, the present disclosure relates to a process for the preparation of the trifluoroacetic acid salt of a compound of formula (I) comprising
i. providing a compound of formula (II) in a solvent or solvent mixture comprising water, acetonitrile, dichloromethane, tetrahydrofurane, 1-methoxy-2-propanol, sulfolane, acetic acid, dimethylacetamide, dimethyl sulfoxide, N-methyl pyrrolidone, dimethyl formamide, 1-propanol, 2-propanol, 1-butanol, 2-butanol, hexafluoro-2-propanol and trifluoroethanol or a mixture of two or more thereof,
ii. reacting the compound of formula (II) with a isothiocyanate-forming reagent to form a compound of formula (I),
iii. purifying the compound of formula (I) by reversed-phase chromatography, wherein at least one eluent comprises trifluoroacetic acid,
iv. isolating the trifluoroacetic acid salt of a compound of formula (I).

### Process for the preparation of the crystalline form of the trifluoroacetic acid salt of a compound of formula (I)

The present disclosure also relates to a process for the preparation of the crystalline form of the trifluoroacetic acid salt of a compound of formula (I) described *supra.*

The process for the preparation of the crystalline form of the trifluoroacetic acid salt of a compound of formula (I) comprises the reaction of a compound of formula (II) with an isothiocyanate-forming reagent in the presence of trifluoroacetic acid.

Any isothiocyanate-forming reagent can be used as long as it is suitable for the conversion of the amino group in the compound of formula (II) into the isothiocyanate group in the compound of formula (I). Preferably, the isothiocyanate-forming group is an aryl or heteroaryl thionocarbonate, an aryl or heteroaryl thiocarbonyl compound or thiophosgene. More preferably, the isothiocyanate-forming group is an aryl or heteroaryl thionocarbonate, thiocarbonyldiimidazol (di-1H-imidazol-1-ylmethanethione), bis(1H-1,2,4-triazol-1-yl)methanethione, bis(1H-benzotriazol-1-yl)methanethione or thiophosgene. More preferably, the isothiocyanate-forming group is bis(2-pyridyl)thionocarbonate, thiocarbonyldiimidazol (di-1H-imidazol-1-ylmethanethione), bis(1H-1,2,4-triazol-1-yl)methanethione, bis(1H-benzotriazol-1-yl)methanethione or thiophosgene. More preferably, the isothiocyanate-forming group is bis(2-pyridyl)thionocarbonate (CAS-No.: 96989-50-3).

The process for the preparation of the crystalline form of the trifluoroacetic acid salt of a compound of formula (I) is preferably carried out in a solvent or solvent mixture comprising an alcohol, preferably in a solvent or solvent mixture comprising an alcohol having at least 3 carbon atoms to avoid the formation of thiocarbamate byproducts resulting from the reaction with the isothiocyanate group in the compound of formula (I). Preferably the process is carried out in a solvent or solvent mixture comprising a C3-C5 alcohol, i.e., an alcohol having three to five carbon atoms. Preferably the process is carried out in a C2-C5 fluorinated alcohol containing at least three fluorine atoms. Preferably the process is carried out in a solvent mixture comprising a C3-C5 alcohol and a C2-C5 fluorinated alcohol containing at least three fluorine atoms. Preferably, the process for the preparation of the crystalline form of the trifluoroacetic acid salt of a compound of formula (I) is carried out in a solvent or solvent mixture comprising an alcohol selected from 1-methoxy-2-propanol, 1-propanol, 2-propanol, 1-butanol, 2-butanol, hexafluoro-2-propanol and trifluoroethanol or a mixture of two or more thereof. More preferably, the process for the preparation of the crystalline form of the trifluoroacetic acid salt of a compound of formula (I) is carried out in 2-propanol or in a mixture of 2-propanol and hexafluoro-2-propanol. More preferably, the process for the preparation of the crystalline form of the trifluoroacetic acid salt of a compound of formula (I) is carried out in a 3:1 (v/v) mixture of 2-propanol and hexafluoro-2-propanol.

The process for the preparation of the crystalline form of the trifluoroacetic acid salt of a compound of formula (I) comprises the use of trifluoroacetic acid. Without being bound by theory and depending on the solvent used for the conversion of the compound of formula (II) to the compound of formula (I), the use of trifluoroacetic acid is presumed to increase the solubility of the starting material, i.e., the compound of formula (II) and, at the same time, to stabilize the compound of formula (I) as a trifluoroacetic acid salt. Preferably, the reaction is carried out using 2 to 10 molar equivalents of trifluoroacetic acid, more preferably using 2 to 8 molar equivalents of trifluoroacetic acid, more preferably using 3 to 5 molar equivalents of trifluoroacetic acid. More preferably, the reaction is carried out using 3 molar equivalents of trifluoroacetic acid.

The process can be carried out at any suitable temperature which leads to the formation of the crystalline form of the trifluoroacetic acid salt of a compound of formula (I). Preferably, the reaction is carried out at a temperature of from 20°C to 50°C, more preferably at a temperature of from 25°C to 45°C.

The temperature of the reaction may be adjusted depending on the amount of trifluoroacetic acid used. Higher temperatures may be needed when larger amounts of trifluoroacetic acid are used. Preferably, the reaction is carried out using 3 molar equivalents of trifluoroacetic acid at a temperature of from 25°C to 30°C or using 8 molar equivalents of trifluoroacetic acid at a temperature of from 35 to 45°C. More preferably, the reaction is carried out using 3 molar equivalents of trifluoroacetic acid at a temperature of from 25°C to 30°C. More preferably, the reaction is carried out using 8 molar equivalents of trifluoroacetic acid at a temperature of from 35 to 45°C.

Preferably, the starting material, i.e., the compound of formula (II) is suspended and/or dissolved in the solvent or solvent mixture in the presence of the corresponding amount of trifluoroacetic acid at a suitable temperature before the isothiocyanate-forming reagent is added.

Upon forming, the crystalline form of the trifluoroacetic acid salt of a compound of formula (I) may precipitate from the reaction mixture as a crystalline solid. Optionally, a further solvent or solvent mixture different from that in which the reaction (i.e., the reaction of a compound of formula (II) with an isothiocyanate-forming reagent in the presence of trifluoroacetic acid) has been carried out may be added once the reaction is complete to induce or complete the crystallization of the crystalline form of the trifluoroacetic acid salt of a compound of formula (I). Preferably, precipitation as a crystalline solid of the trifluoroacetic acid salt of a compound of formula (I) ensues and the further solvent or solvent mixture different from that in which the reaction has been carried out is added to complete the crystallization of the crystalline form of the trifluoroacetic acid salt of a compound of formula (I).

In one preferred embodiment, the reaction is carried out in a solvent or solvent mixture which does not require the addition of a further solvent or solvent mixture to induce or complete the crystallization of the crystalline form of the trifluoroacetic acid salt of a compound of formula (I). Preferably, the reaction is carried out in 2-propanol without the addition of a further solvent or solvent mixture to induce or complete the crystallization of the crystalline form of the trifluoroacetic acid salt of a compound of formula (I).

If a further solvent or solvent mixture different from that in which the reaction has been carried out is added to induce or complete the crystallization of the crystalline form of the trifluoroacetic acid salt of a compound of formula (I), said solvent or solvent mixture preferably comprises a solvent selected from a C3-C5 alcohol, a C5-C7 alkane and an ether or a mixture of two or more thereof. If a further solvent or solvent mixture different from that in which the reaction has been carried out is added to induce or complete the crystallization of the crystalline form of the trifluoroacetic acid salt of a compound of formula (I), said solvent or solvent mixture preferably comprises a solvent selected from 2-propanol, tetrahydrofurane, 2-methyl tetrahydrofurane, methyl tert-butyl ether (MTBE) or a mixture of two or more thereof. If a further solvent or solvent mixture different from that in which the reaction has been carried out is added to induce or complete the crystallization of the crystalline form of the trifluoroacetic acid salt of a compound of formula (I), said solvent or solvent mixture more preferably comprises 2-propanol or methyl tert-butyl ether. More preferably, the reaction is carried out in a solvent mixture comprising a C2-C5 fluorinated alcohol containing at least three fluorine atoms and a further solvent or solvent mixture different from that in which the reaction has been carried out is added to induce or complete the crystallization of the crystalline form of the trifluoroacetic acid salt of a compound of formula (I). More preferably, the reaction is carried out in a solvent mixture comprising a C3-C5 alcohol and a C2-C5 fluorinated alcohol containing at least three fluorine atoms and methyl tert-butyl ether is added to induce or complete the crystallization of the crystalline form of the trifluoroacetic acid salt of a compound of formula (I). More preferably, the reaction is carried out in a solvent mixture comprising a C2-C5 fluorinated alcohol containing at least three fluorine atoms and a further solvent selected from 2-propanol or methyl tert-butyl ether is added to induce or complete the crystallization of the crystalline form of the trifluoroacetic acid salt of a compound of formula (I). More preferably, the reaction is carried out in a mixture of 2-propanol and hexafluoro-2-propanol and a further solvent is added to induce or complete the crystallization of the crystalline form of the trifluoroacetic acid salt of a compound of formula (I). More preferably, the reaction is carried out in a 3:1 (v/v) mixture of 2-propanol and hexafluoro-2-propanol and and methyl tert-butyl ether is added to induce or complete the crystallization of the crystalline form of the trifluoroacetic acid salt of a compound of formula (I).

Optionally, the reaction mixture may be seeded with the crystalline trifluoroacetic acid salt of macropa-(OCH2CH2)-Ph-NCS (i.e., the crystalline trifluoroacetic acid salt of the compound of formula (I)) to induce or complete crystallization of the crystalline form of the trifluoroacetic acid salt of a compound of formula (I).

In one embodiment, the present disclosure relates to a process for the preparation of a crystalline form of the trifluoroacetic acid salt of a compound of formula (I) comprising
i. providing a compound of formula (II) in a solvent or solvent mixture comprising a C3-C5 alcohol,
ii. adding trifluoroacetic acid,
iii. reacting the compound of formula (II) with a isothiocyanate-forming reagent,
iv. isolating the crystalline form of the trifluoroacetic acid salt of a compound of formula (I) from the reaction mixture.

In a further embodiment, the present disclosure relates to a process for the preparation of a crystalline form of the trifluoroacetic acid salt of a compound of formula (I) characterized by having a powder X-ray diffractogram comprising reflections at 2-Theta angles of (6.8 ± 0.2)°, (9.4 ± 0.2)°, (10.1 ± 0.2)°, (11.0 ± 0.2)°, (17.20 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm, said process comprising
i. providing a compound of formula (II) in a solvent or solvent mixture comprising a C3-C5 alcohol,
ii. adding trifluoroacetic acid,
iii. reacting the compound of formula (II) with a isothiocyanate-forming reagent,
iv. isolating the crystalline form of the trifluoroacetic acid salt of a compound of formula (I) from the reaction mixture.

In a further embodiment, the present disclosure relates to a process for the preparation of a crystalline form of the trifluoroacetic acid salt of a compound of formula (I) comprising
i. providing a compound of formula (II) in an alcohol selected from 1-propanol, 2-propanol, 1-butanol, 2-butanol, hexafluoro-2-propanol and trifluoroethanol or a mixture of two or more thereof,
ii. adding 2 to 8 molar equivalents of trifluoroacetic acid,
iii. reacting the compound of formula (II) with a isothiocyanate-forming reagent,
iv. isolating the crystalline form of the trifluoroacetic acid salt of a compound of formula (I) from the reaction mixture.

In a further embodiment, the present disclosure relates to a process for the preparation of a crystalline form of the trifluoroacetic acid salt of a compound of formula (I) characterized by having a powder X-ray diffractogram comprising reflections at 2-Theta angles of (6.8 ± 0.2)°, (9.4 ± 0.2)°, (10.1 ± 0.2)°, (11.0 ± 0.2)°, (17.20 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm, said process comprising
i. providing a compound of formula (II) in an alcohol selected from 1-propanol, 2-propanol, 1-butanol, 2-butanol, hexafluoro-2-propanol and trifluoroethanol or a mixture of two or more thereof,
ii. adding 2 to 8 molar equivalents of trifluoroacetic acid,
iii. reacting the compound of formula (II) with a isothiocyanate-forming reagent,
iv. isolating the crystalline form of the trifluoroacetic acid salt of a compound of formula (I) from the reaction mixture.

In a further embodiment, the present disclosure relates to a process for the preparation of a crystalline form of the trifluoroacetic acid salt of a compound of formula (I) comprising
i. providing a compound of formula (II) in an alcohol selected from 1-propanol, 2-propanol, 1-butanol, 2-butanol, hexafluoro-2-propanol and trifluoroethanol or a mixture of two or more thereof,
ii. adding 2 to 8 molar equivalents of trifluoroacetic acid,
iii. reacting the compound of formula (II) with a isothiocyanate-forming reagent at a temperature of from 20°C to 50°C,
iv. isolating the crystalline form of the trifluoroacetic acid salt of a compound of formula (I) from the reaction mixture.

In a further embodiment, the present disclosure relates to a process for the preparation of a crystalline form of the trifluoroacetic acid salt of a compound of formula (I) characterized by having a powder X-ray diffractogram comprising reflections at 2-Theta angles of (6.8 ± 0.2)°, (9.4 ± 0.2)°, (10.1 ± 0.2)°, (11.0 ± 0.2)°, (17.20 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm, said process comprising
i. providing a compound of formula (II) in an alcohol selected from 1-propanol, 2-propanol, 1-butanol, 2-butanol, hexafluoro-2-propanol and trifluoroethanol or a mixture of two or more thereof,
ii. adding 2 to 8 molar equivalents of trifluoroacetic acid,
iii. reacting the compound of formula (II) with a isothiocyanate-forming reagent at a temperature of from 20°C to 50°C,
iv. isolating the crystalline form of the trifluoroacetic acid salt of a compound of formula (I) from the reaction mixture.

In a further embodiment, the present disclosure relates to a process for the preparation of a crystalline form of the trifluoroacetic acid salt of a compound of formula (I) comprising
i. providing a compound of formula (II) in a solvent mixture comprising a C3-C5 alcohol and a C2-C5 fluorinated alcohol containing at least three fluorine atoms,
ii. adding 2 to 8 molar equivalents of trifluoroacetic acid,
iii. reacting the compound of formula (II) with a isothiocyanate-forming reagent at a temperature of from 20°C to 50°C,
iv. isolating the crystalline form of the trifluoroacetic acid salt of a compound of formula (I) from the reaction mixture.

In a further embodiment, the present disclosure relates to a process for the preparation of a crystalline form of the trifluoroacetic acid salt of a compound of formula (I) characterized by having a powder X-ray diffractogram comprising reflections at 2-Theta angles of (6.8 ± 0.2)°, (9.4 ± 0.2)°, (10.1 ± 0.2)°, (11.0 ± 0.2)°, (17.20 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm, said process comprising
i. providing a compound of formula (II) in a solvent mixture comprising a C3-C5 alcohol and a C2-C5 fluorinated alcohol containing at least three fluorine atoms,
ii. adding 2 to 8 molar equivalents of trifluoroacetic acid,
iii. reacting the compound of formula (II) with a isothiocyanate-forming reagent at a temperature of from 20°C to 50°C,
iv. isolating the crystalline form of the trifluoroacetic acid salt of a compound of formula (I) from the reaction mixture.

In a further embodiment, the present disclosure relates to a process for the preparation of a crystalline form of the trifluoroacetic acid salt of a compound of formula (I) comprising
i. providing a compound of formula (II) in a solvent mixture comprising a C3-C5 alcohol and a C2-C5 fluorinated alcohol containing at least three fluorine atoms,
ii. adding 2 to 8 molar equivalents of trifluoroacetic acid,
iii. reacting the compound of formula (II) with a isothiocyanate-forming reagent at a temperature of from 20°C to 50°C,
iv. isolating the crystalline form of the trifluoroacetic acid salt of a compound of formula (I) from the reaction mixture,
wherein step (iv) comprises the addition of a further solvent or solvent mixture different from that in which the reaction in step (iii) is carried out, said solvent or solvent mixture preferably comprising a solvent selected from 2-propanol, tetrahydrofurane, 2-methyl tetrahydrofurane, methyl tert-butyl ether (MTBE) or a mixture of two or more thereof, more preferably wherein the further solvent is methyl tert-butyl ether (MTBE).

In a further embodiment, the present disclosure relates to a process for the preparation of a crystalline form of the trifluoroacetic acid salt of a compound of formula (I) characterized by having a powder X-ray diffractogram comprising reflections at 2-Theta angles of (6.8 ± 0.2)°, (9.4 ± 0.2)°, (10.1 ± 0.2)°, (11.0 ± 0.2)°, (17.20 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm, said process comprising
i. providing a compound of formula (II) in a solvent mixture comprising a C3-C5 alcohol and a C2-C5 fluorinated alcohol containing at least three fluorine atoms,
ii. adding 2 to 8 molar equivalents of trifluoroacetic acid,
iii. reacting the compound of formula (II) with a isothiocyanate-forming reagent at a temperature of from 20°C to 50°C,
iv. isolating the crystalline form of the trifluoroacetic acid salt of a compound of formula (I) from the reaction mixture,
wherein step (iv) comprises the addition of a further solvent or solvent mixture different from that in which the reaction in step (iii) is carried out, said solvent or solvent mixture preferably comprising a solvent selected from 2-propanol, tetrahydrofurane, 2-methyl tetrahydrofurane, methyl tert-butyl ether (MTBE) or a mixture of two or more thereof, more preferably wherein the further solvent is methyl tert-butyl ether (MTBE).

In a further embodiment, the present disclosure relates to a process for the preparation of a crystalline form of the trifluoroacetic acid salt of a compound of formula (I) comprising
i. providing a compound of formula (II) in an alcohol selected from 1-propanol, 2-propanol, 1-butanol, 2-butanol, hexafluoro-2-propanol and trifluoroethanol or a mixture of two or more thereof,
ii. adding 3 molar equivalents of trifluoroacetic acid,
iii. reacting the compound of formula (II) with a isothiocyanate-forming reagent at a temperature of from 25°C to 30°C,
iv. isolating the crystalline form of the trifluoroacetic acid salt of a compound of formula (I) from the reaction mixture.

In a further embodiment, the present disclosure relates to a process for the preparation of a crystalline form of the trifluoroacetic acid salt of a compound of formula (I) characterized by having a powder X-ray diffractogram comprising reflections at 2-Theta angles of (6.8 ± 0.2)°, (9.4 ± 0.2)°, (10.1 ± 0.2)°, (11.0 ± 0.2)°, (17.20 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm, said process comprising
i. providing a compound of formula (II) in an alcohol selected from 1-propanol, 2-propanol, 1-butanol, 2-butanol, hexafluoro-2-propanol and trifluoroethanol or a mixture of two or more thereof,
ii. adding 3 molar equivalents of trifluoroacetic acid,
iii. reacting the compound of formula (II) with a isothiocyanate-forming reagent at a temperature of from 25°C to 30°C,
iv. isolating the crystalline form of the trifluoroacetic acid salt of a compound of formula (I) from the reaction mixture.

In a further embodiment, the present disclosure relates to a process for the preparation of a crystalline form of the trifluoroacetic acid salt of a compound of formula (I) comprising
i. providing a compound of formula (II) in an alcohol selected from 1-propanol, 2-propanol, 1-butanol, 2-butanol, hexafluoro-2-propanol and trifluoroethanol or a mixture of two or more thereof,
ii. adding 2 to 8 molar equivalents of trifluoroacetic acid,
iii. reacting the compound of formula (II) with a isothiocyanate-forming reagent selected from an aryl or heteroaryl thionocarbonate, thiocarbonyldiimidazol (di-1H-imidazol-1-ylmethanethione), bis(1H-1,2,4-triazol-1-yl)methanethione, bis(1H-benzotriazol-1-yl)methanethione and thiophosgene, preferably wherein the isothiocyanate-forming group is selected from bis(2-pyridyl)thionocarbonate, thiocarbonyldiimidazol (di-1H-imidazol-1-ylmethanethione), bis(1H-1,2,4-triazol-1-yl)methanethione, bis(1H-benzotriazol-1-yl)methanethione and thiophosgene at a temperature of from 20°C to 50°C,
iv. isolating the crystalline form of the trifluoroacetic acid salt of a compound of formula (I) from the reaction mixture.

In a further embodiment, the present disclosure relates to a process for the preparation of a crystalline form of the trifluoroacetic acid salt of a compound of formula (I) characterized by having a powder X-ray diffractogram comprising reflections at 2-Theta angles of (6.8 ± 0.2)°, (9.4 ± 0.2)°, (10.1 ± 0.2)°, (11.0 ± 0.2)°, (17.20 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm, said process comprising
i. providing a compound of formula (II) in an alcohol selected from 1-propanol, 2-propanol, 1-butanol, 2-butanol, hexafluoro-2-propanol and trifluoroethanol or a mixture of two or more thereof,
ii. adding 2 to 8 molar equivalents of trifluoroacetic acid,
iii. reacting the compound of formula (II) with a isothiocyanate-forming reagent selected from an aryl or heteroaryl thionocarbonate, thiocarbonyldiimidazol (di-1H-imidazol-1-ylmethanethione), bis(1H-1,2,4-triazol-1-yl)methanethione, bis(1H-benzotriazol-1-yl)methanethione and thiophosgene, preferably wherein the isothiocyanate-forming group is selected from bis(2-pyridyl)thionocarbonate, thiocarbonyldiimidazol (di-1H-imidazol-1-ylmethanethione), bis(1H-1,2,4-triazol-1-yl)methanethione, bis(1H-benzotriazol-1-yl)methanethione and thiophosgene at a temperature of from 20°C to 50°C,
iv. isolating the crystalline form of the trifluoroacetic acid salt of a compound of formula (I) from the reaction mixture.

In a further embodiment, the present disclosure relates to a process for the preparation of a crystalline form of the trifluoroacetic acid salt of a compound of formula (I) comprising
i. providing a compound of formula (II) in a 3:1 (v/v) mixture of 2-propanol and hexafluoro-2-propanol.
ii. adding 2 to 8 molar equivalents of trifluoroacetic acid,
iii. reacting the compound of formula (II) with a isothiocyanate-forming reagent at a temperature of from 20°C to 50°C,
iv. isolating the crystalline form of the trifluoroacetic acid salt of a compound of formula (I) from the reaction mixture.

In a further embodiment, the present disclosure relates to a process for the preparation of a crystalline form of the trifluoroacetic acid salt of a compound of formula (I) characterized by having a powder X-ray diffractogram comprising reflections at 2-Theta angles of (6.8 ± 0.2)°, (9.4 ± 0.2)°, (10.1 ± 0.2)°, (11.0 ± 0.2)°, (17.20 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm, said process comprising
i. providing a compound of formula (II) in a 3:1 (v/v) mixture of 2-propanol and hexafluoro-2-propanol.
ii. adding 2 to 8 molar equivalents of trifluoroacetic acid,
iii. reacting the compound of formula (II) with a isothiocyanate-forming reagent at a temperature of from 20°C to 50°C,
iv. isolating the crystalline form of the trifluoroacetic acid salt of a compound of formula (I) from the reaction mixture.

It is to be understood that the present invention relates also to any combination of the embodiments described above.

The present disclosure covers methods of preparing compounds of the present disclosure, said methods comprising the steps as described in the Experimental Section herein.

In a further embodiment, the present disclosure covers the use of a compound of formula (II) for the preparation of a trifluoroacetic acid salt of a compound of formula (I).

In a further embodiment, the present disclosure covers the use of a compound of formula (II) for the preparation of a crystalline form of the trifluoroacetic acid salt of a compound of formula (I).

In a further embodiment, the present disclosure covers the use of a compound of formula (II) for the preparation of a crystalline form of the trifluoroacetic acid salt of a compound of formula (I) characterized by having a powder X-ray diffractogram comprising reflections at 2-Theta angles of (6.8 ± 0.2)°, (9.4 ± 0.2)°, (10.1 ± 0.2)°, (11.0 ± 0.2)°, (17.20 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm.

In a further embodiment, the present disclosure covers the use of the trifluoroacetic acid salt of a compound of formula (I) and/or the use of the the crystalline form of the trifluoroacetic acid salt of a compound of formula (I) for the preparation of a conjugate.

In a further embodiment, the present disclosure covers the use of the trifluoroacetic acid salt of a compound of formula (I) and/or the use of the the crystalline form of the trifluoroacetic acid salt of a compound of formula (I) for the preparation of a conjugate of formula (III) 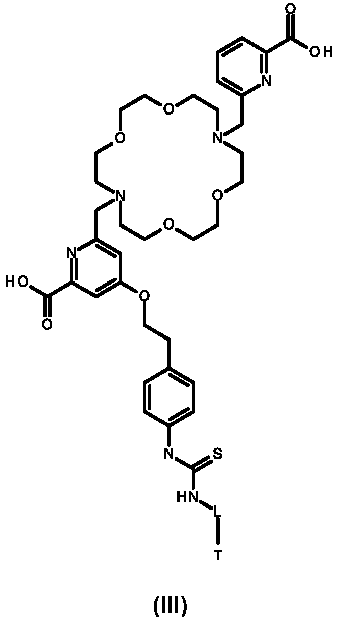 wherein L is an optional linking moiety and T is a targeting moiety.

In a further embodiment, the present disclosure covers the use of the trifluoroacetic acid salt of a compound of formula (I) and/or the use of the the crystalline form of the trifluoroacetic acid salt of a compound of formula (I) characterized by having a powder X-ray diffractogram comprising reflections at 2-Theta angles of (6.8 ± 0.2)°, (9.4 ± 0.2)°, (10.1 ± 0.2)°, (11.0 ± 0.2)°, (17.20 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm for the preparation of a conjugate of formula (III) 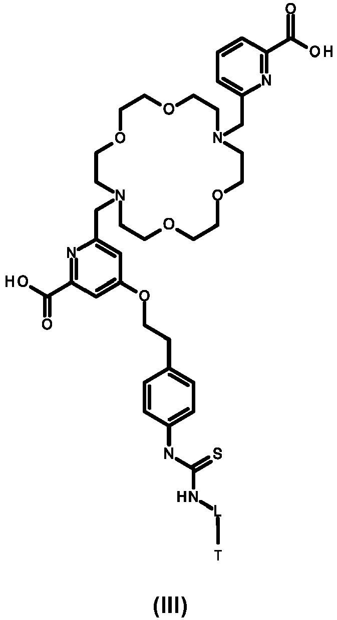 wherein L is an optional linking moiety and T is a targeting moiety.

In a further embodiment, the present disclosure covers the use of the trifluoroacetic acid salt of a compound of formula (I) and/or the use of the the crystalline form of the trifluoroacetic acid salt of a compound of formula (I) characterized by having a powder X-ray diffractogram comprising reflections at 2-Theta angles of (6.8 ±0.2)°, (9.4 ± 0.2)°, (10.1 ± 0.2)°, (11.0 ± 0.2)°, (17.20 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm for the preparation of a conjugate of formula (III) 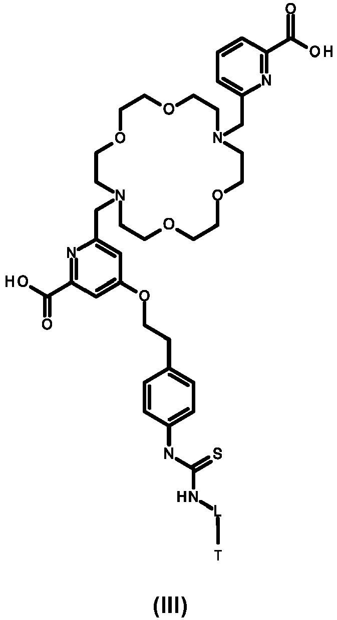 wherein L is an optional linking moiety and T is a targeting moiety comprising an antibody, an antibody fragment (e.g., an antigen-binding fragment), a binding moiety, a binding peptide, a binding polypeptide (such as a selective targeting oligopeptide containing up to 50 amino acids), a binding protein, an enzyme, a nucleobase-containing moiety (such as an oligonucleotide, DNA or RNA vector, or aptamer), or a lectin.

In a further embodiment, the present disclosure covers the use of the trifluoroacetic acid salt of a compound of formula (I) and/or the use of the the crystalline form of the trifluoroacetic acid salt of a compound of formula (I) characterized by having a powder X-ray diffractogram comprising reflections at 2-Theta angles of (6.8 ± 0.2)°, (9.4 ± 0.2)°, (10.1 ± 0.2)°, (11.0 ± 0.2)°, (17.20 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm for the preparation of a conjugate of formula (III) 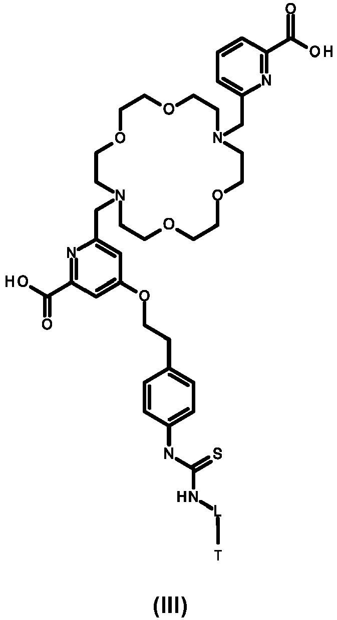 wherein L is an optional linking moiety and T is a targeting moiety selected from an antibody, an antibody fragment (e.g., an antigen-binding fragment), a binding moiety, a binding peptide, a binding polypeptide (such as a selective targeting oligopeptide containing up to 50 amino acids), a binding protein, an enzyme, a nucleobase-containing moiety (such as an oligonucleotide, DNA or RNA vector, or aptamer), or a lectin.

In a further embodiment, the present disclosure covers the use of the trifluoroacetic acid salt of a compound of formula (I) and/or the use of the the crystalline form of the trifluoroacetic acid salt of a compound of formula (I) characterized by having a powder X-ray diffractogram comprising reflections at 2-Theta angles of (6.8 ± 0.2)°, (9.4 ± 0.2)°, (10.1 ± 0.2)°, (11.0 ± 0.2)°, (17.20 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm for the preparation of a conjugate of formula (Illa) 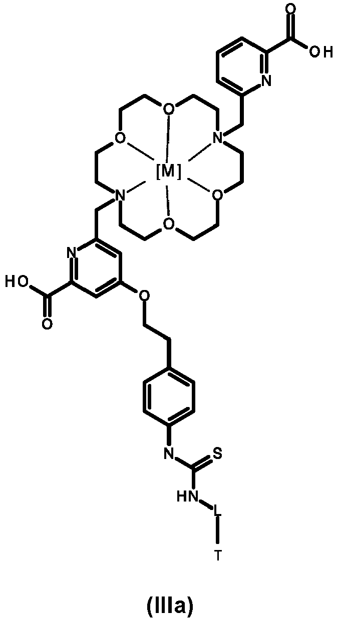 wherein L is an optional linking moiety and T is a targeting moiety selected from an antibody, an antibody fragment (e.g., an antigen-binding fragment), a binding moiety, a binding peptide, a binding polypeptide (such as a selective targeting oligopeptide containing up to 50 amino acids), a binding protein, an enzyme, a nucleobase-containing moiety (such as an oligonucleotide, DNA or RNA vector, or aptamer), or a lectin and [M] is a radiation-emitting radionuclide, preferably an alpha-emitting radionuclide, preferably an alpha-emitting radionuclide selected from actinium-225 (225Ac3+), radium-223, (223Ra2+), bismuth-213 (213Bi3+), lead-212 (212Pb2+ and/or 212Pb4+), terbium-149 (149Tb3+), fermium-255 (255Fm3+), thorium-227 (227Th4+), thorium-226 (226Th4+), astatine-211 (211At+), astatine-217 (217At+), or uranium-230, more preferably 225Ac3+.

### DESCRIPTION OF THE FIGURES

Figure 1 displays the powder X-ray diffractogram (XRD) of two samples of the crystalline form of the trifluoroacetic acid salt of the compound of formula (I) macropa-(OCH2CH2)-Ph-NCS prepared according to Examples 2d (top) and 2a (bottom).
Figure 2 displays the DSC (top) and TGA (bottom) curves of a sample of the crystalline form of the trifluoroacetic acid salt of the compound of formula (I) macropa-(OCH2CH2)-Ph-NCS prepared according to Example 2a.
Figure 3 displays the DSC (top) and TGA (bottom) curves of a sample of the crystalline form of the trifluoroacetic acid salt of the compound of formula (I) macropa-(OCH2CH2)-Ph-NCS prepared according to Example 2d.
Figure 4 displays the infrared spectrum of a sample of the crystalline form of the trifluoroacetic acid salt of the compound of formula (I) macropa-(OCH2CH2)-Ph-NCS prepared according to Example 2a.

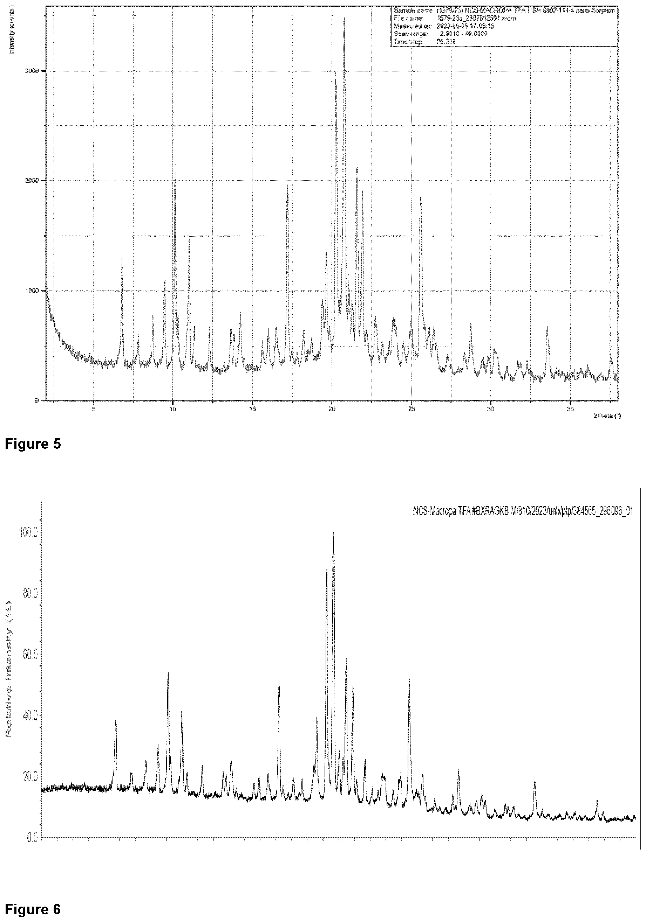

Figure 5 displays the powder X-ray diffractogram (XRD) of the crystalline form of the trifluoroacetic acid salt of the compound of formula (I) macropa-(OCH2CH2)-Ph-NCS prepared according to Example 2b.
Figure 6 displays the powder X-ray diffractogram (XRD) of the crystalline form of the trifluoroacetic acid salt of the compound of formula (I) macropa-(OCH2CH2)-Ph-NCS prepared according to Example 2c.

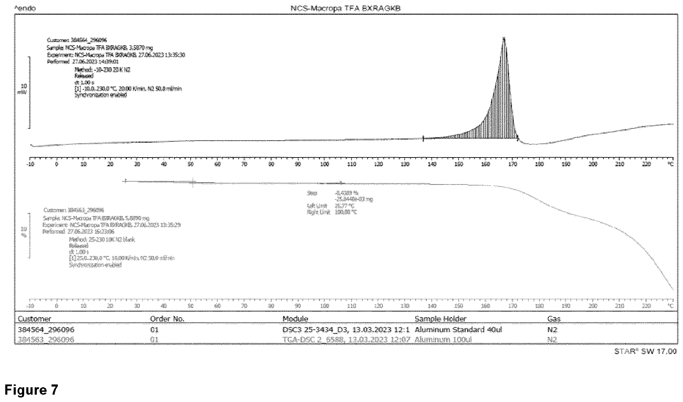

Figure 7 displays the DSC (top) and TGA (bottom) curves of a sample of the crystalline form of the trifluoroacetic acid salt of the compound of formula (I) macropa-(OCH2CH2)-Ph-NCS prepared according to Example 2c.

### EXPERIMENTAL SECTION

Chemical names were generated using the ACD/Name software from ACD/Labs. In some cases generally accepted names of commercially available reagents were used in place of ACD/Name generated names.

The following table 1 lists the abbreviations used in this paragraph and in the Examples section as far as they are not explained within the text body. Other abbreviations have their meanings customary *per se* to the skilled person.

**Table 1. Abbreviations**

| **Abbreviation** | **Meaning** |
|---|---|
| AcOH | acetic acid |
| ACN | acetonitrile |
| aq. | aqueous |
| br | broad signal (NMR) |
| d | doublet (NMR) |
| DAD | Diode Array Detector |
| DCM | Dichloromethane |
| dd | doublet of doublet (NMR) |
| DIPEA | diisopropylethylamine |
| DMA | *N,N*-dimethylacetamide |
| DMF | *N,N*-dimethylformamide |
| DMSO | Dimethylsulfoxide |
| ESI | electrospray (ES) ionization |
| EtOAc | ethyl acetate |
| EtOH | ethanol |
| h | hour(s) |
| HFIP | 1,1,1,3,3,3-Hexafluoro-2-propanol |
| HCl | hydrogen chloride, hydrochloric acid |
| HPLC | high performance liquid chromatography |
| ICP-MS | inductively coupled plasma mass spectrometry |
| IR | Infra Red |
| LC-MS | liquid chromatography-mass spectrometry |
| m | multiplet (NMR) |
| MeCN | Acetonitrile |
| MeOH | Methanol |
| min | minute(s) |
| MS | mass spectrometry |
| MTBE | Methyl-*tert*-butylether |
| MWD | multiple wavelength detector |
| NMR | Nuclear Magnetic Resonance spectroscopy : chemical shifts (δ) are given in ppm. The chemical shifts were corrected by setting the DMSO signal to 2.50 ppm using unless otherwise stated. |
| q | quartet (NMR) |
| Rt or RT | room temperature |
| Rₜ, Rt | retention time |
| s | singulet (NMR) |
| sat. | saturated |
| SFC | supercritical fluid chromatography |
| t | triplet (NMR) |
| td | triplet of doublet (NMR) |
| TEA | triethylamine |
| TFA | trifluoroacetic acid |
| TFE | trifluoroethanol |
| THF | tetrahydrofuran |
| δ | chemical shift |

Other abbreviations have their meanings customary *per se* to the skilled person.

The various aspects of the invention described in this application are illustrated by the following examples which are not meant to limit the invention in any way.

The example testing experiments described herein serve to illustrate the present invention and the invention is not limited to the examples given.

### Experimental section - general part

All reagents for which the synthesis is not described in the experimental part, are either commercially available, or are known compounds or may be formed from known compounds by known methods by a person skilled in the art.

macropa-(OCH2CH2)-Ph-NH2 was prepared according to the synthesis described in WO2020/106886.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be removed by trituration using a suitable solvent. In some cases, the compounds may be purified by chromatography.

In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

### NMR Spectra

The multiplicities of proton signals in ¹H NMR spectra given in the following paragraphs reflect the observed signal form and do not take into account any higher-order signal phenomena. As a rule, the chemical shift data refers to the center of the signal in question. In the case of wide multiplets, a range is specified. Signals hidden by solvent or water were either assigned tentatively or are not listed. Strongly broadened signals - *e.g.* caused by rapid rotation of molecular moieties or by interchanging protons - have also been assigned tentatively (often referred to as a broad multiplet or broad singlet) or are not shown.

The ¹H-NMR data of selected compounds are listed in the form of ¹H-NMR peaklists. Therein, for each signal peak the δ value in ppm is given, followed by the signal intensity, reported in round brackets. The δ value-signal intensity pairs from different peaks are separated by commas. Therefore, a peaklist is described by the general form: δ₁ (intensity,), δ₂ (intensity₂), ... , δᵢ (intensityᵢ), ... , δₙ (intensityₙ).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A ¹H-NMR peaklist is similar to a classical ¹H-NMR readout, and thus usually contains all the peaks listed in a classical NMR interpretation. Moreover, similar to classical ¹H-NMR printouts, peaklists can show solvent signals, signals derived from stereoisomers of the particular target compound, peaks of impurities, ¹³C satellite peaks, and/or spinning sidebands. The peaks of stereoisomers, and/or peaks of impurities are typically displayed with a lower intensity compared to the peaks of the target compound (e.g., with a purity of >90%). Such stereoisomers and/or impurities may be typical for the particular manufacturing process, and therefore their peaks may help to identify a reproduction of the manufacturing process on the basis of "by-product fingerprints". An expert who calculates the peaks of the target compound by known methods (MestReC, ACD simulation, or by use of empirically evaluated expectation values), can isolate the peaks of the target compound as required, optionally using additional intensity filters. Such an operation would be similar to peak-picking in classical ¹H-NMR interpretation. A detailed description of the reporting of NMR data in the form of peaklists can be found in the publication "Citation of NMR Peaklist Data within Patent Applications" (cf. http://www.researchdisclosure.com/searching-disclosures, Research Disclosure Database Number 605005, 2014, 01 Aug 2014). In the peak picking routine, as described in the Research Disclosure Database Number 605005, the parameter "MinimumHeight" can be adjusted between 1% and 4%. However, depending on the chemical structure and/or depending on the concentration of the measured compound it may be reasonable to set the parameter "MinimumHeight" <1%.

### Analytical Methods

### LC-MS

| | |
|---|---|
| Method 1: | System MS: Waters TOF instrument; System UPLC: Waters Acquity I-CLASS; Column: Waters Acquity UPLC HSS T3, 2.1 x 150 mm, 1.8 µm; Eluent A: 1 L Water + 0.100 mL 99% trifluoroacetic acid, Eluent B: 1 L Acetonitrile + 0.100 |
| | mL 99% trifluoroacetic acid; Gradient: 0.0 min 5% B → 1 min 5% B → 13 min 95% B → 15 min 95% B; Oven: 50°C; Flow: 0.60 mL/min; UV-Detection: 210 nm. |

### HPLC

| | |
|---|---|
| Method 2a: | System UPLC: Agilent 1290 Infinity I; Column: Waters Acquity HSS Peptide T3 C18; 2.1 x 100 mm, 1.8 µm; Eluent A: 0.1% TFA in water; Eluent B: 0.085% TFA in acetonitrile; Gradient: 0.0 min → 2.0 min 8% B → 12.0 min 50% B → 15.0 min 50% B → 15.1 min 80% B → 18.0 min 80% B → 18.1 min 8% B → 23.0 min 8% B; Oven: 50°C; Flow: 0.8 mL/min; UV-Detection 225 nm. |
| Method 2b: | System UPLC: Agilent 1290 Infinity I; Column: Waters Acquity HSS Peptide T3 C18; 2.1 x 100 mm, 1.8 µm; Eluent A: 0.1% TFA in water; Eluent B: 0.085% TFA in acetonitrile; Gradient: 0.0 min → 2.0 min 0% B → 12.0 min 50% B → 15.0 min 50% B → 15.1 min 80% B → 18.0 min 80% B → 18.1 min 0% B → 23.0 min 0% B; Oven: 60°C; Flow: 0.9 mL/min; UV-Detection 225 nm. |
| Method 3a: | System HPLC: Agilent 1100; Column: Agilent Zorbax SB-Aq; 3.0 x 150 mm, 3.5 µm; Eluent A: Water; Eluent B: Acetonitrile; Gradient: 0.0 min 5% B → 20.0 min 80% B; Oven: 45°C; Flow: 0.5 mL/min; UV-Detection 210 nm. |
| Method 3b: | System HPLC: Agilent 1260 Infinity; Column: Agilent Zorbax SB-Aq; 3.0 x 150 mm, 3.5 µm; Eluent A: Water; Eluent B: Acetonitrile; Gradient: 0.0 min 5% B → 20.0 min 80% B; Oven: 45°C; Flow: 0.5 mL/min; UV-Detection 210 nm. |

### XRD

X-ray powder diffraction (XRPD) data were recorded on a STOE STADI P diffractometer using monochromatized CuK _{α₁}-radiation, a position sensitive detector, at generator settings of 40 kV and 40 mA. The samples were collected in transition mode, being prepared as a thin layer between two foils. The scanning range was between 2 ° and 40 ° 2 theta with a 0.5° step at 15 sec/step.

### DSC

Differential scanning calorimetry (DSC) was performed with a Mettler Toledo DSC3. The calorimeter was purged with nitrogen gas at a flow rate of 50 ml.min-1. 3 - 10 mg of each sample was placed into an aluminum crucible and heated at a rate of 20 °C.min -1 from -10 °C to either 230°C, or to 20 - 30 K above the highest melting point or to the beginning of decomposition.

### TGA

Thermogravimetric analysis (TGA) was performed with a Mettler Toledo TGA/DSC 3+. The instrument was purged with nitrogen gas at a flow rate of 50 ml.min-1. Approximately 5 - 8 mg of each sample was placed into an aluminum crucible with piercing lid and heated at a heating rate of 10 °C.min -1 from 25 °C to 20 - 30 K above the highest melting point or to the beginning of decomposition.

### IR

IR measurements were performed with a Bruker alpha spectrometer in the attenuated total reflectance (ATR) geometry. No sample preparation was performed, and each individual measurement consisted of 32 scans.

### DVS

Water sorption isotherms were determined using a Hiden IGAsorp gravimetric sorption analyzer. 10 - 20 mg of sample was equilibrated at 0 % RH/25°C and the weight recorded. The isotherm was then recorded by changing the humidity in steps of 10 % and 5 %. The equilibrium criterion was set to a relative mass change of dm/dt = 0.0005 mg/min.

### Synthesis of the Examples

### Example 1: Preparation of the amorphous trifluoroacetic salt of macropa-(OCH2CH2)-Ph-NCS (trifluoroacetic acid salt of the compound of formula (I)

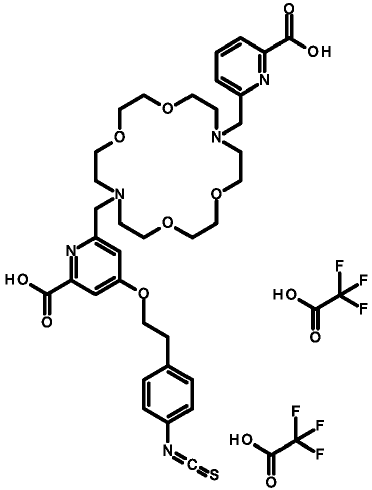

3.00 g (4.49 mmol) macropa-(OCH2CH2)-Ph-NH2 was suspended in 24 mL acetonitrile. After addition of 7.2 ml water a turbid solution formed. After addition of 1.15 g (4.94 mmol) di-2-pyridyl thionocarbonate (O,O-dipyridin-2-yl carbonothioate) at room temperature a clear solution formed. HPLC control (method 3a) confirmed complete conversion after 10 min. 90 mL water was added and the reaction mixture was purified by reversed phase chromatography at 400 g Kromasil C18-100-16 with stepwise change of the eluent composition:
400 mL fractions - eluent A: water + 0.1% TFA; eluent B: acetonitrile (Table 2)

**Table 2**

| Fraction | Eluent A | Eluent B |
|---|---|---|
| 1 | 80.0 | 20.0 |
| 2 | 77.5 | 22.5 |
| 3 | 75.0 | 25.0 |
| 4 | 72.5 | 27.5 |
| 5 | 70.0 | 30.0 |
| 6 | 67.5 | 32.5 |
| 7 | 65.0 | 35.0 |
| 8 | 62.5 | 37.5 |
| 9 | 60.0 | 40.0 |
| 10 | 57.5 | 57.5 |
| 11 | 20.0 | 80.0 |
| 12 | 20.0 | 80.0 |

Product containing fractions (5b¹, 6 and 7) were combined and immediately freeze-dried.
¹ Fraction 5 was split into 2 sub-fractions a and b to separate from impurities.

4.0 g (95 % of theory) of lyophilisate was isolated.

5 batches were produced at 3 g-scale and with one re-purification batch of mixed fractions combined by dissolution in water and freeze-drying to obtain a homogenized batch of 19.10 g product (91 % of theoretical value) as a nearly white lyophilisate.
Purity by HPLC, method 2a: 99.2 area-%; Rt: 10.02 min
Assay free macropa-(OCH2CH2)-Ph-NCS: 69.1 wt.-%
TFA by ion-chromatography: 28.10 wt.-% (theoretical value: 24.3 wt.-%)
Stability study (Table 3)

**Table 3.**

| | Start | 6 months, 5°C |
|---|---|---|
| Assay free base (wt.-% | 69.06 | 67.26 |
| HPLC-purity (area-%) | 99.09 | 98.16 |
| Water content (wt.-%) | 0.83 | 1.10 |

### Example 2: Preparation of the crystalline trifluoroacetic salt of macropa-(OCH2CH2)-Ph-NCS (crystalline trifluoroacetic acid salt of the compound of formula (I)

### Example 2a

80.0 g (0.12 mol) of macropa-(OCH2CH2)-Ph-NH2 was suspended in 0.80 L 2-propanol and 73.8 mL trifluoroacetic acid (0.96 mol) was added. The mixture was warmed to 40°C to give a solution after 10 min. 36.2 g (0.16 mol) di-2-pyridyl thionocarbonate was added to the mixture and stirring was continued overnight. The resulting suspension was cooled to room temperature and diluted with 0.80 mL methyl-tert-butyl ether (MTBE). The product was isolated by filtration. The filter cake was rinsed 4 times with 80 mL MTBE/2-propanol (1:1 v/v), each. After drying in vacuum at 30°C 92.5 g (82 % of theoretical yield) was obtained as a white to off-white solid.

| | |
|---|---|
| Purity by HPLC, method 2a: | 96.7 area-%; Rt: 9.33 min |
| Impurity at RRT: 0.97², method 2a: | 0.59 area-%; Rₜ: 9.10 min |
| Assay "free macropa-(OCH2CH2)-Ph-NCS": | 73.8 wt.-% |
| TFA by ion-chromatography: | 21.8 wt.-% (theoretical value: 24.3 wt.-%) -- measured at re-release. |

| | |
|---|---|
| ¹H-NMR (D₂O; 500 MHz) δ [ppm] = 3.13 (m, 2H), 3.58 (m, 4H), 3.63 (m, 12 H), 3.89 (m, 8H), 4.47 (m, 2H), 4.63 (s, 2H), 4.74 (s, *), 7.18 (m, 2H)**, 7.23 (m, 1H), 7.33 (m, 2H), 7.47 (m, 1H), 7.71 (m, 1H), 7.98 (m, 1H), 8.08 (m, 1H). * Significant overlap with the signal resulting from measurement in deuterium-oxide. ² Identified later as macropa-(OCH2CH2)-phenyl-2-propyl-thiocarbamate. ** A shift of this 2H-signal of the phenylene-moiety is observed between batches. The assumed root-cause is a different protonation status of the NCS-group under TFA-acidic conditions in D₂O. XRPD (2Theta): 6.8°, 9.4°, 10.1°, 11.0°, 17.2°, 20.2°, 20.7°, 21.5°, 21.9°, 25.5°, Figure 1 (top). DSC/TGA: Figure 2 IR: Figure 4 | |

DVS analysis confirmed the stability of the sample product (crystalline trifluoroacetic salt of macropa-(OCH2CH2)-Ph-NCS, i.e., the crystalline trifluoroacetic acid salt of the compound of formula (I)).

LC-HRMS (method 1): Rt = 5.70 min (98 %); MS (ESlpos): m/z = 710.2883 (M+H)⁺.

### Stability study (Table 4)

**Table 4**

| | Start | 6 months, 5°C |
|---|---|---|
| Assay free base (wt.-%) | 73.81 | 72.72 |
| HPLC-purity (area-%) | 96.82 | 96.62 |
| Water content (wt.-%) | 0.43 | 0.41 |

### Example 2b

Following the crystallization process of Example 2a from 5.0 g macropa-(OCH2CH2)-Ph-NH2 6.5 g (93 % of theoretical yield) of the crystalline trifluoroacetic acid salt of a compound of formula (I) was obtained.

XRPD analysis confirmed the same crystalline form as in Example 2a.

| | |
|---|---|
| Purity by HPLC, method 2a: | 96.9 area-%; Rt: 9.42 min |
| Impurity at RRT: 0.97³, method 2a: | 0.57 area-%; Rₜ: 9.18 min |
| Assay "free macropa-(OCH2CH2)-Ph-NCS": | 71.7 wt.-% |
| TFA by ion-chromatography: | 22.3 wt.-% |

| | |
|---|---|
| ³ Identified later as macropa-(OCH2CH2)-phenyl-2-propyl-thiocarbamate. | |

The ¹NMR showed the 2H-signal of the phenylene-moiety located at 7.24 ppm.

¹H-NMR (D₂O; 500 MHz) δ [ppm] = 3.15 (t, 2H), 3.54 (m, 4H), 3.61 (m, 12 H), 3.87 (m, 8H), 4.50 (t, 2H*), 4.60 (s, 2H*), 4.72 (s, 2H*), 7.22 (m, 1H), 7.24 (m, 2H), 7.36 (m, 2H), 7.51 (m, 1H), 7.70 (m, 1H), 8.01 (m, 1H), 8.07 (m, 1H).

### Example 2c

The process outlined above (Example 2a, Example 2b) was repeated in a GMP-laboratory in a larger scale.

A glass-reactor was charged with 137.2 g (92.7 mL; 1.20 mol) trifluoroacetic acid and 788 g (1.0 L) 2-propanol and 100 g (0.15 mol) macropa-(OCH2CH2)-Ph-NH2 was added. The suspension was heated to 38°C and an almost clear solution formed. 44.8 g (0.19 mol) di-2-pyridyl thionocarbonate was added and stirred at 38°C overnight (22:30 h). The reaction mixture was cooled to 22°C and diluted with 740 g (1.0 L) MTBE. After 12 min at 19°C the product was isolated by filtration. The filter cake was rinsed 4 times with MTBE/2-propanol (1:1 v/v) approx. 100 mL each and dried in vacuum at 30°C.

123 g (88 % of theoretical yield) of the crystalline product was obtained as a light-yellow solid.

| | |
|---|---|
| Purity by HPLC, method 2a: | 94.7 area-%, Rₜ: 9.46 min |
| Impurity at RRT: 0.97⁴, method 2a: | 2.50 area-%; Rₜ: 9.20 min |
| Assay "free macropa-(OCH2CH2)-Ph-NCS": | 69.7 wt.-% |
| TFA by ion-chromatography: | 23.7 wt.-% (theoretical value 24.3 wt.-%). |
| Main impurity*: | 2.5 area-%, RT = 9.20 min |

| | |
|---|---|
| ⁴ Identified as macropa-(OCH2CH2)-phenyl -2-propyl-thiocarbamate. | |

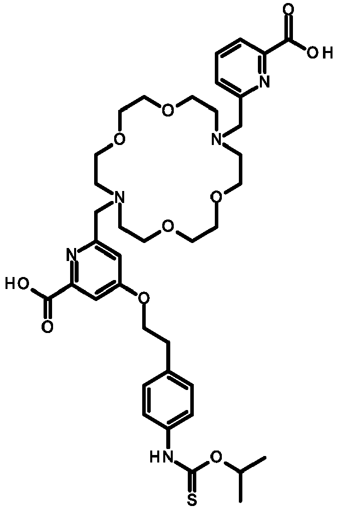

XRPD and DSC/TGA analysis confirmed the crystalline form of the product (i.e., the crystalline trifluoroacetic acid salt of the compound of formula (I)).

LC-HRMS (method 1): Rt = 5.80 min (89 %); MS (ESlpos): m/z = 710.2866 (M+H)⁺.

### Example 2d

To a mixture of 752 mL (590 g) 2-propanol and 253 mL (403 g) hexafluoro-2-propanol (HFIP) was added 51.2 g (34.6 mL, 0.45 mol) trifluoroacetic acid and 100 g (0.15 mol) macropa-(OCH2CH2)-Ph-NH2. The mixture was stirred at 23°C and an almost clear solution was formed. 52.2 g (0.22 mol) di-2-pyridyl thionocarbonate was added, followed by addition of 0.7 g (0.7 mmol) seed crystals of crystalline macropa-(OCH2CH2)-Ph-NCS bis TFA salt. The reaction mixture was stirred at 23°C for 6:20 h. 1.11 kg (1.5 L) MTBE was added, and the suspension was stirred at 20°C overnight, i.e. 17:30 h. The product was isolated by filtration. The filter cake was rinsed three times with MTBE/2-propanol (3:2 v/v) approx. 125 mL each and once with 93 g (125 mL) MTBE. The product was dried in vacuum at 30°C. 133 g (95 % of theoretical value) of crystalline, white product was obtained.

| | |
|---|---|
| Purity by HPLC, method 2b | 98.8 area-%; Rₜ: 9.36 min |
| Impurity at RRT: 0.98⁵, method 2b: | 0.14 area-%; Rₜ: 9.16 min |
| Assay "free macropa-(OCH2CH2)-Ph-NCS": | 74.2 wt.-% |
| ⁵ Identified as macropa-(OCH2CH2)-phenyl -2-propyl-thiocarbamate. | |
| TFA by ion-chromatography: | 23.3 wt.-% (theoretical value 24.3 wt.-%). |

| | |
|---|---|
| DSC-TGA: Figure 3 | |

¹H-NMR (D₂O; 500 MHz) δ [ppm] = 3.11 (m, 2H), 3.58 (m, 4H), 3.63 (m, 12 H), 3.90 (m, 8H), 4.45 (m), 4.64 (s), 4.75 (s), 7.14 (m, 2H), 7.23 (m, 1H), 7.31 (m, 2H), 7.45 (m, 1H), 7.72 (m, 1H), 7.98 (m, 1H), 8.06 (m, 1H).

### Example 2e

A series of experiments with varying equivalents of the activation reagent were performed:
50 mg (0.075 mmol) macropa-(OCH2CH2)-Ph-NH2 was suspended in 0.5 mL 2-propanol and 46 µL (0.6 mmol) trifluoroacetic acid was added. The mixture was warmed to 40°C and a clear solution formed after 10 min. The corresponding amount of di-2-pyridyl thionocarbonate was added (s. table) followed by seeding with a small amount of crystalline macropa-(OCH2CH2)-Ph-NCS bis TFA salt. The mixture was stirred at 40°C overnight and the results are summarized in Table 5.

**Table 5**

| **Experiment** | **eq. Activation reagent** | **HPLC after 20h at 40°C** | |
|---|---|---|---|
| | | **area-% product** | **Area-% macropa-(OCH2CH2)-Ph-NH2** |
| 2e-1 | 1,00 | 85,5 | 1,7 |
| 2e-2 | 1,10 | 85,6 | 2,1 |
| 2e-3 | 1,15 | 85,2 | 1,6 |
| 2e-4 | 1,20 | 82,9 | 1,5 |
| 2e-5 | 1,25 | 84,2 | 1,3 |
| 2e-6 | 1,30 | 85,4 | 1,4 |
| 2e-7 | 1,60 | 81,9 | 0,6 |
| 2e-8 | 3,00 | 66,9 | n.n. |

| | | | |
|---|---|---|---|
| HPLC method: 3a | | | |

### Example 2f - Effect of different amounts of trifluoroacetic acid:

50 mg (0.075 mmol) macropa-(OCH2CH2)-Ph-NH2 was suspended in 0.5 mL 2-propanol and various amounts of trifluoroacetic acid were added (see table below). The mixtures were stirred at room temperature and after 30 min gave clear solutions. 35 mg (0.15 mmol) di-2-pyridyl thionocarbonate was added followed by seeding with a small amount of crystalline macropa-(OCH2CH2)-Ph-NCS bis TFA salt. The reaction mixtures were stirred at room temperature overnight (20h). The results of the in-process controls by HPLC are summarized in Table 6.

**Table 6**

| **Example** | **Sample** | **eq. TFA** | **Reaction-Time** | **Product area-%** | **Starting material area-%** | **Byproduct Rt: 13.5/13.6 min. area-%** |
|---|---|---|---|---|---|---|
| 2f-1 | a | 3 | 2 | 78,3 | n.d. | 0,04 |
| | a | 3 | 20 | 78,3 | 0,3 | 0,03 |
| 2f-2 | b | 4 | 2 | 76,8 | 2,6 | 0,02 |
| | b | 4 | 20 | 77,5 | 0,5 | 0,03 |
| 2f-3 | c | 4 | 2 | 79,4 | 0,1 | 0,05 |
| | c | 4 | 20 | 78,5 | 0,7 | 0,07 |
| 2f-4 | d | 5 | 2 | 79,2 | 1,2 | 0,05 |
| | d | 5 | 20 | 77,9 | 1,7 | 0,09 |
| 2f-5 | e | 8 | 2 | 73,0 | 5,7 | 0,05 |
| | e | 8 | 20 | 77,9 | 1,2 | 0,14 |
| 2f-6 | f | 16 | 2 | 66,8 | 11,0 | 0,36 |
| | f | 16 | 20 | 77,1 | 1,7 | 0,70 |

The byproduct at Rₜ: 13.5/13.6 min was identified as a mixture of the two regioisomeric mono-2-propyl-esters of product macropa-(OCH2CH2)-Ph-NCS.

### HPLC method: 3a

### Example 2q - Effect of different solvents for the preparation of the crystalline trifluoroacetic salt of a compound of formula (I):

50 mg scale; 0.5 mL solvent, room temperature; 3 eq. TFA; 1.5 eq thionocarbonate reagent 50 mg (0.075 mmol) macropa-(OCH2CH2)-Ph-NH2 was suspended each in 0.5 mL various solvents (see table below) and 17 µL (0.23 mmol) trifluoroacetic acid was added to each reaction mixture. The mixtures were stirred at room temperature and after 1 h gave clear solutions except for 2-methyl-1-propanol where a small quantity of undissolved material remained. 26 mg (0.11 mmol) di-2-pyridyl thionocarbonate was added to each reaction mixture which were all clear solutions followed by seeding with a small amount of crystalline macropa-(OCH2CH2)-Ph-NCS bis TFA salt which did not dissolve. The reaction mixtures were stirred at room temperature overnight (20h) and precipitation occured. The results of the in-process controls by HPLC are summarized in Table 7.

**Table 7**

| **Example** | **Sample** | **Solvent** | **Reaction Time** | **Product** | **Starting Material** | **Byproduct Bis-macropa thiourea** |
|---|---|---|---|---|---|---|
| | | | **[h]** | **Area-%** | **Area-%** | **Area-%** |
| 2e-1 | a | n-Propanol | 2 | 82,7 | 1,6 | 0,3 |
| | a | | 20 | 81,2 | 1,3 | 0,3 |
| 2e-2 | b | 2-Methyl-1-propanol | 2 | 83,3 | 1,2 | 0,6 |
| | b | | 20 | 84,6 | 1,1 | 0,4 |
| 2e-3 | c | 1-Methoxy-2-propanol | 2 | 82,7 | 0,2 | 1,6 |
| | c | | 20 | 83,3 | 0,04 | 1,5 |
| 2e-4 | d | 2-propanol/ HFIP (3:1 (v/v)) | 2 | 83,2 | 0,6 | 0,2 |
| | d | | 20 | 82,9 | 1,2 | 0,1 |

| | | | | | | |
|---|---|---|---|---|---|---|
| HPLC method: 3a | | | | | | |

0.5 mL MTBE was added to each of the reaction mixtures and stirring was continued at room temperature for 1 h. The product was isolated by filtration. The filter cake was rinsed two times with 0.25 mL of 2-propanol/MTBE (1:1 v/v) each. After drying in vacuum at 30°C the product was obtained in the yields and HPLC-purities depicted in Table 8.

**Table 8**

| **Example** | **Sample** | **Solvent** | **Yield** | **Yield** | **Product** | **Starting Material** | **Byproduct Bis-macropa thiourea** |
|---|---|---|---|---|---|---|---|
| | | | **[mg]** | **[%]** | **[Area-%]** | **[Area-%]** | **[Area-%]** |
| 2e-1 | a | n-Propanol | 50 | 71 | 97.2 | 1.9 | 0.4 |
| 2e-2 | b | 2-Methyl-1-propanol | 52 | 74 | 97.0 | 1.5 | 0.5 |
| 2e-3 | c | 1-Methoxy-2-propanol | 50 | 71 | 97.7 | 0.1 | 1.8 |
| 2e-4 | d | 2-propanol/ HFIP | 46 | 66 | 96.7 | 2.5 | 0.2 |

| | | | | | | | |
|---|---|---|---|---|---|---|---|
| HPLC-controls: Method 3a. | | | | | | | |

### Example 2h - Solvent effects in the (attempted) preparation of the crystalline trifluoroacetic salt of a compound of formula (I)

### 1. Methanol

To 50 mg (0.075 mmol) macropa-(OCH2CH2)-Ph-NH2 was added 0.5 mL methanol and 46 µL (0.6 mmol) trifluoroacetic acid. The mixture was warmed to 40°C and a clear solution formed. 23 mg (0.10 mmol) di-2-pyridyl thionocarbonate was added and a small amount of seeds of crystalline macropa-(OCH2CH2)-Ph-NCS bis TFA salt was added. The seeds dissolved. The reaction mixture was stirred overnight (20h) at 40°C and remained a clear solution. HPLC control (method: 3a) revealed a complex mixture with 40.6 area-% product, 5.6 area-% bis-macropa-thiourea and an unknown impurity with 29.9 area-%. The latter was identified subsequently as the corresponding methanol-adduct, i.e. the macropa-(OCH2CH2)-phenyl-methyl-thiocarbamate.

### 2. Ethanol

To 50 mg (0.075 mmol) macropa-(OCH2CH2)-Ph-NH2 was added 0.5 mL ethanol and 46 µL (0.6 mmol) trifluoroacetic acid. The mixture was warmed to 40°C and a clear solution formed. 23 mg (0.10 mmol) di-2-pyridyl thionocarbonate was added and a small amount of seeds of crystalline macropa-(OCH2CH2)-Ph-NCS bis TFA salt was added. The seeds dissolved. The reaction mixture was stirred overnight (20h) at 40°C and remained a clear solution. HPLC control (method 3a) revealed a complex mixture with 56.7 area-% product, 0.9 area-% bis-macropa-thiourea and an unknown impurity with 17.3 area-%. The latter was identified subsequently as the corresponding ethanol-adduct, i.e. the macropa-(OCH2CH2)-phenylethyl-thiocarbamate.

### 3. Dimethylacetamide

20 mg (0.02 mmol) macropa-(OCH2CH2)-Ph-NH2 tris trifluoroacetate was dissolved in 0.16 mL DMA. 4.6 mg (0.02 mmol) di-2-pyridyl thionocarbonate was added and the reaction solution was stirred at room temperature overnight.

HPLC controls (method 3a) after 10 min, 30 min and overnight reaction time revealed incomplete conversion with approximately 54 - 57 area-% of product and a significant amount of symmetrical bis-macropa-thiourea byproduct formed besides other impurities.

### 4. Mixtures of 2-propanol and dimethylacetamide (DMA)

50 mg (0.075 mmol) macropa-(OCH2CH2)-Ph-NH2 was dissolved in 0.5 mL of the following solvent mixtures:
0.45 mL 2-propanol and 0.05 mL DMA (sample a, Example 2h-1)
0.40 mL 2-propanol and 0.10 mL DMA (sample b, Example 2h-2)
0.35 mL 2-propanol and 0.15 mL DMA (sample c, Example 2h-3)

To each solution 17 µL (0.225 mmol) trifluoroacetic acid was added. After stirring for 30 min at room temperature 26 mg (0.112 mmol) di-2-pyridyl thionocarbonate, each, was added to the clear solutions, directly followed by seeding with a small amount of crystalline macropa-(OCH2CH2)-Ph-NCS bis TFA salt. The seeds did not dissolve. The reaction mixtures were stirred overnight at room temperature.

0.5 mL MTBE was added to each suspension and stirring was continued for 1 h at room temperature. The product was isolated by filtration, two rinses with 0.25 mL of 2-propanol/MTBE (1:1 v/v), each and drying in vacuum at 30°C.

Slightly beige coloured products were obtained (Table 9).

**Table 9**

| Example | Sample | Yield | Yield | Product | Starting Material | Byproduct Rt 7.7 min | 2-Propanol adduct |
|---|---|---|---|---|---|---|---|
| | | [mg] | [%] | [Area-%] | [Area-%] | [Area-%] | [Area-%] |
| 2h-1 | a | 52 | 74 | 97.5 | 1.3 | 0.5 | 0.14 |
| 2h-2 | b | 46 | 66 | 97.1 | 0.2 | 1.2 | 0.33 |
| 2h-3 | c | 14 | 20 | 96.1 | 0.1 | 1.0 | 0.60 |

| | | | | | | | |
|---|---|---|---|---|---|---|---|
| HPLC-method: 2b | | | | | | | |

### 5. Hexafluoro-2-propanol

200 mg (0.30 mmol) macropa-(OCH2CH2)-Ph-NH2 was dissolved in 2.0 mL HFIP. 76.5 mg (0.33 mmol) di-2-pyridyl thionocarbonate was added and the reaction mixture was stirred at room temperature. The HPLC control after 2:30 h showed 87 area-% of product. 5 mL 2-propanol and 185 µL (2.40 mmol) trifluoroacetic acid were added and the mixture was concentrated at 40°C and 60 mbar to a volume of approximately 2 mL. 5 mL 2-propanol was added and the volume reduced to 2 mL a second time. The solution was cooled to 0-5°C and seed crystals of the product were added. After stirring for 2 h at this temperature a thin suspension had formed. Additional 92 µL (1.2 mmol) trifluoroacetic acid was added and the mixture was stored at 2 - 8°C overnight. The precipitate was isolated by filtration and the filter-cake was rinsed twice with 0.5 mL 2-propanol each. After drying in vacuum at 30°C 175 mg (62 % of theory) of crystalline product macropa-(OCH2CH2)-Ph-NCS bis TFA salt was obtained. The XRPD confirmed the same polymorphic form as described above.

### 6. THF

To 20 mg (0.03 mmol) macropa-(OCH2CH2)-Ph-NH2 was added 0.20 mL THF and 18.5 µL (0.24 mmol) trifluoroacetic acid. After sonication for 10 min a solution formed. 7.7 mg (0.033 mmol) di-2-pyridyl thionocarbonate was added and the reaction mixture was stirred at room temperature overnight. The HPLC control (method 3a) showed 86 area-% of product, 0.4 area-% of starting material and 3.2 area-% of bis-macropa-thiourea (HPLC method 3a). By addition of 0.2 mL MTBE the product was precipitated. However, due to the sticky resinous consistency isolation was not pursued.

### Example 2i - Conversion of the amorphous trifluoroacetic salt of a compound of formula (I) (i.e., amorphous macropa-(OCH2CH2)-Ph-NCS) to the crystalline trifluoroacetic salt of a compound of formula (I) (i.e, amorphous macropa-(OCH2CH2)-Ph-NCS) by intermediary dissolution by addition of triethylamine

500 mg (0.53 mmol) macropa-(OCH2CH2)-Ph-NCS bis TFA (lyophilisate) was suspended in 5.0 mL 2-propanol and turned into a sticky consistency. The mixture was heated to 50°C and an oily residue was observed. Upon addition of 0.30 mL (2.13 mmol) triethylamine and stirring for 2 h at 40°C a solution formed. 0.16 mL (2.13 mmol) trifluoroacetic acid was added and the mixture was cooled to 0°C within 2 h and precipitation occurred. After filtration, two rinses with 0.5 mL 2-propanol each and drying in vacuum at 30°C 427 mg (85 % of theory) of a crystalline white solid was obtained. The XRPD was in agreement with the polymorphic form described above. HPLC (method 3a): 97.1 area-%.

### Example 2i - Alternative Acids

The following table summarizes a series of experiments using alternative acids to trifluoroacetic acid. The generic procedure was:
The starting material macropa-(OCH2CH2)-Ph-NH2 was suspended in the solvent and the corresponding acid was added. The mixture was stirred at the given temperature and the activation reagent di-2-pyridyl thionocarbonate was added. The reaction-mixture was stirred at the same temperature overnight. An in-process control by HPLC was performed and the appearance of the reaction mixture was controlled (Table 10).

| **Table 10** | | | | | | | | |
|---|---|---|---|---|---|---|---|---|
| Experiment | Acid | Amount SM | Amount Acid | Solvent | Temp. | Amount Reagent | Reac-tion Time | Result |
| 2j-1 | Methanesulfonic acid | | | 2-propanol 0.2 mL | 40°C | | overnight | 29.6 % P |
| | | 20 mg | 15.5 µL | | | 9.0 mg | | 21.7 %SM |
| | | 0.03 mmol | 0.24 mmol | | | 0.04 mmol | | BPs; Sol. |
| 2j-2 | 5M HCl in 2-propanol | | | | 40°C | | overnight | 42.2 % P |
| | | 20 mg | 47.9 µL | 2- | | 9.0 mg | | 2.8 % SM |
| | | 0.03 mmol | 0.24 mmol | propanol 0.2 mL | | 0.04 mmol | | BPs X |
| 2j-3 | Oxalic acid | | | 2-propanol 0.2 mL | 40°C | | overnight | 63.9 % P |
| | | 20 mg | 21.6 mg | | | 9.0 mg | | 1.0 % SM |
| | | 0.03 mmol | 0.24 mmol | | | 0.04 mmol | | BPs X |
| 2j-4 | KH₂PO₄ | | | 2-propanol 0.2 mL | 40°C | | overnight | 38.5 % P |
| | | 20 mg | 32.6 mg | | | 9.0 mg | | 0 % SM |
| | | 0.03 mmol | 0.24 mmol | | | 0.04 mmol | | BPs X |
| 2j-5 | Formic acid | | | 2-propanol 0.2 mL | 40°C | | overnight | 1.1 % P |
| | | 20 mg | 9.0 µL | | | 9.0 mg | | 1.5 % SM |
| | | 0.03 mmol | 0.24 mmol | | | 0.04 mmol | | BPs* X |
| 2j-6 | Methanesulfonic acid | | | 2-propanol 0.2 mL | 40°C | | overnight | 74.0 % P |
| | | 20 mg | 5.8 µL | | | 9.0 mg | | 0.2 % SM |
| | | 0.03 mmol | 0.09 mmol | | | 0.04 mmol | | BPs Y |
| 2j-7 | Citric acid | 20 mg | 17.2 mg | 2-propanol 0.2 mL | | 9.0 mg | overnight | X |
| | | 0.03 mmol | 0.09 mmol | | 40°C | 0.04 mmol | | |
| 2j-8 | Citric acid mono hydrate | 20 mg | 18.9 mg | 2-propanol 0.2 mL | | 9.0 mg | overnight | X |
| | | 0.03 mmol | 0.09 mmol | | 40°C | 0.04 mmol | | |
| 2j-9 | 4-toluenesulfonic acid | | | 2-propanol 2.0 mL | | | overnight | 4.4 % P |
| | | 200 mg | 456 | | | 90.4 mg | | BPs |
| | | 0.30 mmol | mg 2.4 mmol | | 40°C | 0.39 mmol | | Sol. Z |
| 2j-10 | Benzenesulfonic acid | | | 2-propanol 2.0 mL | | | overnight | 0.6 % P |
| | | 200 mg | 379 mg | | | 90.4 mg | | BPs |
| | | 0.30 mmol | 2.4 mmol | | 40°C | 0.39 mmol | | Sol. Z |
| 2j-11 | Methanesulfonic acid | | | 2-propanol 2.0 mL | | | overnight | 1.1 % P |
| | | 200 mg | 155 µL | | | 90.4 mg | | PBs |
| | | 0.30 mmol | 2.4 mmol | | 40°C | 0.39 mmol | | Sol. Z |
| 2j-12 | Oxalic acid | 200 mg | 216 mg | 2-propanol | | 90.4 mg | overnight | 65.5 % P |
| | | 0.30 mmol | 2.4 mmol | | 40°C | 0.39 mmol | | BPs Z |
| 2j-13 | 85% H₃PO₄ | 200 mg | 148 µL | 2-propanol | | 90.4 mg | overnight | 49.8 % P |
| | Phosphor ic acid | 0.30 mmol | 2.4 mmol | | 40°C | 0.39 mmol | | BPs Z |
| 2j-14 | Ethane-disulfonic acid | 200 mg | 228 mg | 2-propanol | | 90.4 mg | overnight | < 0.5 % P |
| | | 0.30 mmol | 2.4 mmol | | 40°C | 0.39 mmol | | BPs |
| 2j-15 | Sulfamic acid | 100 mg | 116 mg | 2-propanol | 40°C | 45.2 mg | overnight | 45.2 % P |
| | | 0.15 mmol | 1.2 mmol | | | 0.20 mmol | | BPs X |
| 2j-16 | Sacchari n | | | 2-propanol | 40°C | | overnight | 14.7 % P |
| | | 100 mg | 219 mg | | | 45.2 mg | | 15.9 % |
| | | 0.15 mmol | 1.2 mmol | | | 0.20 mmol | | P** BPs X |
| 2j-17 | Diphenyl-phosphini c acid | | | 2-propanol | 40°C | | overnight | 23.9 % P |
| | | 100 mg | 261 mg | | | 45.2 mg | | 27.0 % |
| | | 0.15 mmol | 1.2 mmol | | | 0.20 mmol | | P** BPs X |
| 2j-18 | Phenyl-phosphon ic acid | | | 2-propanol | 40°C | | overnight | 20.2 % P |
| | | 100 mg | 189 mg | | | 45.2 mg | | 77.6 % |
| | | 0.15 mmol | 1.2 mmol | | | 0.20 mmol | | P** BPs X |
| 2j-19 | Acetic acid | 50 mg | 34.3 µL | 2-propanol | 40°C | 22.6 mg | overnight | 15.6 % P |
| | | 0.075 mmol | 0.6 mmol | | | 0.10 mmol | | BPs X |
| 2j-20 | Fumaric acid | 50 mg | 69.5 mg | 2-propanol | 40°C | 22.6 mg | overnight | 6.7 % P |
| | | 0.075 mmol | 0.6 mmol | | | 0.10 mmol | | BPs X |
| 2j-21 | Succinic acid | 50 mg | 70.7 mg | 2-propanol | 40°C | 22.6 mg | overnight | 47.5 % P |
| | | 0.075 mmol | 0.6 mmol | | | 0.10 mmol | | BPs X |
| 2j-22 | Benzoic acid | 50 mg 0.075 | 73.2 mg | 2-propanol | 40°C | 22.6 mg 0.10 | overnight | 20.7 % P BPs |
| | | mmol | 0.6 mmol | | | mmol | | X |
| 2j-23 | Oxalic acid | 20 mg | 2.7 mg | 1-propanol(0.2 mL) | RT | 10 mg | overnight | Solution, no precipitat e |
| | | 0.03 mmol | 0.03 mmol | HFIP (0.085 mL) | | 0.045 mmol | | |
| | | | | | | | | |
| 2j-24 | Formic acid | 20 mg | 100 µL | 1-propanol (0.2 mL) | RT | 10 mg | overnight | Solution, no precipitat e |
| | | 0.03 | | | | 0.045 | | |
| | | mmol | 2.6 mmol | | | mmol | | |
| 2j-25 | Trichloroacetic acid | 100 mg | 196 mg | 2-propanol | 40°C | 45 mg | overnight | 75.0 % P |
| | | 0.15 mmol | 1.2 mmol | | | 0.20 mmol | | BPs X |
| 2j-26 | Armstron g's acid (Napthali n-1,5-disulfonic acid) | 100 mg | 431 mg | 2-propanol | 40°C | 45 mg | overnight | < 1 % P X |
| | | 0.15 mmol | 1.2 mmol | | | 0.20 mmol | | |
| 2j-27 | Trichloroacetic acid | 50 mg | 98 mg | acetonitril e | 40°C | 23 mg | overnight | 32.4 % P |
| | | 0.075 mmol | 0.6 mmol | | | 0.10 mmol | | BPs X |
| 2j-28 | Trichloroacetic acid | 50 mg | 98 mg | Dichloromethane | 40°C | 23 mg | overnight | 54.3 % P BPs |
| | | 0.075 mmol | 0.6 mmol | | | 0.10 mmol | | Solution, no precipitat e |

| | | | | | | | | |
|---|---|---|---|---|---|---|---|---|
| Sol: solution, i.e., no (solid) precipitate P: product, area-% HPLC in-process control (method 3a) P1: product, area-% HPLC in-process control (method 2b) SM: starting material, area-% in-process control (method 3a) BPs: significant amounts of byproducts X: Consistence not suitable for isolation (sticky, resinous, oily) Y: suspension; work-up: addition of 0.5 mL MTBE, filtration, 2 rinses with 0.05 mL MTBE/2-propanol (1:1, v/v): non-crystalline solid, 94.0 area-% product; 1.4 area-% starting material Z: addition of 2.0 mL MTBE and seeding led to "oiling out". * main component: formamide of the starting material. ** by LC-MS method 1. | | | | | | | | |

## Claims

1. The trifluoroacetic acid salt of a compound of formula (I),

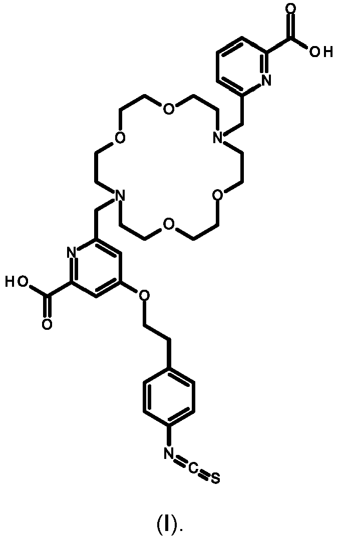

2. The trifluoroacetic salt of a compound of formula (I) according to claim 1, comprising two molar equivalents of trifluoroacetic acid per molar equivalent of the compound of formula (I).

3. The trifluoroacetic salt of a compound of formula (I) according to any of claims 1 or 2 in crystalline form having a powder X-ray diffractogram comprising reflections at 2-Theta angles of (10.1 ± 0.2)°, (20.7 ± 0.2)° and (25.5 ± 0.2)°, when measured at room temperature with Cu-Kalpha1 radiation having a wavelength of 0.15419 nm.

4. The crystalline trifluoroacetic salt of a compound of formula (I) according to claim 3 comprising reflections at 2-Theta angles of (6.8 ± 0.2)°, (10.1 ± 0.2)°, (17.2 ± 0.2)°, (20.2 ± 0.2)°, (20.7 ± 0.2)°, (21.5 ± 0.2)° and (25.5 ± 0.2)°, preferably comprising reflections at 2-Theta angles of (6.8 ± 0.2)°, (9.4 ± 0.2)°, (10.1 ± 0.2)°, (11.0 ± 0.2)°, (17.2 ± 0.2)°, (20.2 ± 0.2)°, (20.7 ± 0.2)°, (21.5 ± 0.2)°, (21.9 ± 0.2)° and (25.5 ± 0.2)°, when measured at room temperature with Cu-Kalpha1 radiation having a wavelength of 0.15419 nm.

5. A process for the preparation of the trifluoroacetic salt of a compound of formula (I) according to any of claims 1 or 2 comprising
i. providing a compound of formula (II) in a solvent or solvent mixture comprising water, acetonitrile, dichloromethane, tetrahydrofurane, 1-methoxy-2-propanol, sulfolane, acetic acid, dimethylacetamide, dimethyl sulfoxide, N-methyl pyrrolidone, dimethyl formamide, 1-propanol, 2-propanol, 1-butanol, 2-butanol, hexafluoro-2-propanol and trifluoroethanol or a mixture of two or more thereof, ii. reacting the compound of formula (II) with a isothiocyanate-forming reagent to form a compound of formula (I),
iii. purifying the compound of formula (I) by reversed-phase chromatography, wherein at least one eluent comprises trifluoroacetic acid,
iv. isolating the trifluoroacetic acid salt of a compound of formula (I).

6. The process according to claim 5, wherein the solvent in step (i) is a mixture of water and acetonitrile.

7. A process for the preparation of a crystalline form of the trifluoroacetic acid salt of a compound of formula (I) according to any of claims 3 or 4 comprising
i. providing a compound of formula (II) in a solvent or solvent mixture comprising a C3-C5 alcohol,
ii. adding trifluoroacetic acid,
iii. reacting the compound of formula (II) with a isothiocyanate-forming reagent,
iv. isolating the crystalline form of the trifluoroacetic acid salt of a compound of formula (I) from the reaction mixture.

8. The process according to claim 7, wherein the solvent or solvent mixture in step (i) comprises a C3-C5 alcohol selected from 1-propanol, 2-propanol, 1-butanol and 2-butanol and a C2-C5 fluorinated alcohol containing at least three fluorine atoms.

9. The process according to any of claims 7 or 8, wherein the solvent or solvent mixture in step (i) comprises a C3-C5 alcohol selected from 1-propanol, 2-propanol, 1-butanol and 2-butanol and a C2-C5 fluorinated alcohol containing at least three fluorine atoms selected from hexafluoro-2-propanol and trifluoroethanol.

10. The process according to any of claims 7 to 9, wherein the solvent or solvent mixture in step (i)) is a 3:1 (v/v) mixture of 2-propanol and hexafluoro-2-propanol.

11. The process according to any of claims 7 to 10, wherein 2 to 8 molar equivalents of trifluoroacetic acid are added in step (ii) and/or wherein the reaction in step (iii) is carried out at a temperature of from 20 to 50°C.

12. The process according to any of claims 7 to 11, wherein 3 molar equivalents of trifluoroacetic acid are added in step (ii) and wherein the reaction in step (iii) is carried out at a temperature of from 25 to 30°C.

13. The process according to any of claims 7 to 11, wherein 8 molar equivalents of trifluoroacetic acid are added in step (ii) and wherein the reaction in step (iii) is carried out at a temperature of from 35 to 45°C.

14. The process according to any of claims 7 to 13, wherein step (iv) comprises the addition of a further solvent or solvent mixture different from that in which the reaction in step (iii) is carried out, said solvent or solvent mixture preferably comprising a solvent selected from 2-propanol, tetrahydrofurane, 2-methyl tetrahydrofurane, methyl tert-butyl ether (MTBE) or a mixture of two or more thereof, more preferably wherein the further solvent is methyl tert-butyl ether (MTBE).

15. Use of the trifluoroacetic acid salt of a compound of formula (I) according to any of claim 1 or 2 and/or use of the the crystalline form of the trifluoroacetic acid salt of a compound of formula (I) according to any of claims 3 or 4 for the preparation of a conjugate of formula (III) 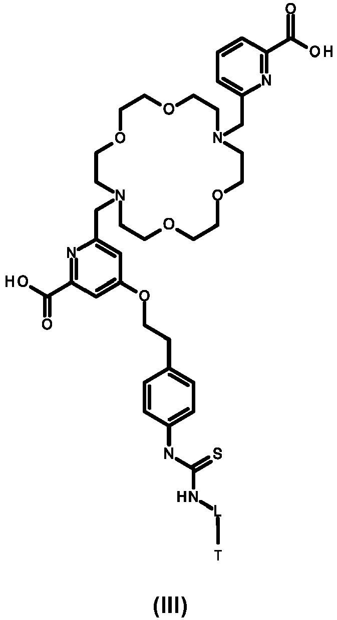 wherein L is an optional linking moiety and T is a targeting moiety.
